# EUROPEAN PATENT APPLICATION

(11) **EP 2 949 326 A2**
(43) Date of publication of application: **02.12.2015**
(21) Application number: 14743024.3
(22) Date of filing: 25.01.2014
(51) Int. Cl.: A61K 31/395, A61K 31/5377, A61K 45/00, A61P 35/00, A61P 43/00

(54) **COMBINATION MOLECULARLY TARGETED DRUG FOR TUMOR THERAPY AND PREVENTION**

(30) Priority: 25.01.2013 JP 2013012705
(71) Applicant: Sakai, Toshiyuki, Kyoto 606-0957 (JP); Oncolys BioPharma, Inc., Tokyo 105-0001 (JP)
(72) Inventor: SAKAI, Toshiyuki, Kyoto-shi Kyoto 606-0957 (JP)
(74) Representative: V.O.
(86) International application number: PCT/JP2014/051589
(87) International publication number: WO 2014/115859

(57) **Abstract**

To provide means for treating and preventing a wide variety of cancers and tumors, including cancer in which there is PI3K/Akt pathway activation or p53 deactivation, or tumors for which chemotherapy, radiation therapy, hormone therapy, and other such conventional treatment methods have low effectiveness, different molecularly targeted drugs such as OBP-801 and PI3K inhibitor-preferably LY294002, BKM120, GDC-0941, BEZ235, BYL719, or CH5132799-are used in combination. This makes it possible to simultaneously obtain a plurality of different marked pharmaceutical benefits which are synergistic, not being obtainable through use of a formulation with either of the respective molecularly targeted drugs alone, such as caspase pathway activation, enhanced expression of Bim, increased accumulation of intracellular reactive oxygen species, and suppressed expression of +survivin and XIAP protein, and makes it possible to provide new and clinically effective tumor treatment/prevention strategies in the form of effective therapeutic agents and prophylactic agents capable of application to a wide variety of tumors.

## Description

### TECHNICAL FIELD

The present invention relates to molecularly targeted combination therapeutic and preventive methods in the context of tumor treatment and prevention.

### BACKGROUND ART

Cancer and other such malignant tumors constitute a principal cause of death among deaths caused by disease. While there have been increases in effectiveness of chemotherapy in which chemotherapeutic agents are employed on tumors, clinical treatment outcomes remain less than satisfactory. For example, morbidity in endometrial cancer worldwide is 150,000 persons per year (see Nonpatent Reference No. 1), and because there has been increase in the number of cases in which there is recurrence of disease and the number of cases in which there is progression of disease in accompaniment to the recent marked increase in morbidity rate, development of effective treatment methods has become a topic for study, and attempts have been made to develop new anticancer agents (see Nonpatent Reference Nos. 2 through 5). Furthermore, renal cell carcinoma constitutes 2% to 3% of adult malignant tumors, and accounts for 12,000 deaths per year worldwide (see Nonpatent Reference Nos. 6 through 8).

There are still malignant tumors such as cancer or the like which cannot be adequately dealt with using existing treatment methods such as the foregoing. Development of various molecularly targeted drugs for overcoming such malignant tumors is underway. Histone deacetylase (HDAC) inhibitors are molecularly targeted drugs, some of which have been reported to possess activity with respect to induction of carcinoma cell growth inhibition, cell differentiation, and apoptosis (see Nonpatent Reference Nos. 9 through 10). The present inventors have found that, by acting on molecules downstream from p53 to cause recovery of p53 functionality, HDAC inhibitors exhibit antitumor effect with respect to malignant tumors possessing p53 mutations (see Nonpatent Reference Nos. 11 through 17). Furthermore, as a result of screening of p21^{WAF1/Cip1}-inducing agents, the present inventors have identified OBP-801/YM753 (spiruchostatin A) as a novel HDAC inhibitor (see Nonpatent Reference No. 18, Patent Reference No. 1, and Patent Reference No. 2). However, except for the example of approval of use of SAHA/vorinostat and romidepsin to treat cutaneous T-cell lymphoma, there is no report of an HDAC inhibitor which has been approved for use in treatment of cancer or other such malignant tumors in a clinical context.

Furthermore, with respect to endometrial cancer, while there have been reports that the phosphatidylinositol 3-kinase (PI3K)/Akt pathway is activated in accompaniment to mutation of PI3K and PTEN (phosphatase and tensin homolog deleted on chromosome 10) (see Nonpatent Reference No. 19), that PI3K inhibitors in the form of molecularly targeted drugs, possess action by which growth of cancer cells carrying the aforementioned mutations is suppressed (see Nonpatent Reference Nos. 20 through 21), and that PI3K inhibitors inhibit cell growth and induce apoptosis of cells in a plurality of malignant tumors including renal cell carcinoma (see Nonpatent Reference Nos. 22 through 26), there is no report of approval for use of a PI3K inhibitor which is a molecularly targeted drug for cancer or other such malignant tumors in a clinical context.

Furthermore, while the multi-tyrosine kinase inhibitors sorafenib and sunitinib, the mammalian target of rapamycin (mTOR) inhibitors everolimus and temsirolimus, and other such molecularly targeted drugs have been developed for renal cell carcinoma and the like, occurrence of drug resistance has been an issue during treatment (see Nonpatent Reference Nos. 27 through 29).

Thus, as it cannot be said that adequate results have been achieved despite the various attempts which have been made, there is still urgent need for development of new treatment strategies to improve clinical outcomes in treatment and prevention of cancer and other such malignant tumors and for development of new therapeutic agents based on said treatment strategies.

### PRIOR ART REFERENCES

### PATENT REFERENCES

Patent Reference No. 1: Japanese Patent No. 3554707
Patent Reference No. 2: Japanese Patent Application Publication Kokai No. 2001-348340

### NONPATENT REFERENCES

Nonpatent Reference No. 1: Okuda T, Sekizawa A, Purwosunu Y, et al. Genetics of endometrial cancers. Obstet Gynecol Int 2010; 2010: 984013
Nonpatent Reference No. 2: Amant F, Moerman P, Neven P, et al. Endometrial cancer. Lancet 2005; 366: 491-505.
Nonpatent Reference No. 3: Rose PG. Endometrial carcinoma. N Engl J Med 1996; 335: 64 0-9.
Nonpatent Reference No. 4: Salvesen HB, Haldorsen IS, Trovik J, Markers for individualised therapy in endometrial carcinoma. Lancet Oncol 2012; 13: 353-61 .
Nonpatent Reference No. 5: Dedes KJ, Wetterskog D, Ashworth A, et al. Emerging therapeutic targets in endometrial cancer. Nat Rev Clin Oncol 2011; 8: 261-71.
Nonpatent Reference No. 6: Rini BI, Campbell SC, Escudier B. Renal cell carcinoma. Lancet. 2009; 373: 1119-32.
Nonpatent Reference No. 7: Gupta K, Miller JD, Li JZ, Russell MW, Charbonneau C. Epidemiologic and socioeconomic burden of metastatic renal cell carcinoma (mRCC): a literature review. Cancer treatment reviews. 2008; 34: 1 93-205.
Nonpatent Reference No. 8: Gupta K, Miller JD, Li JZ, Russell MW, Charbonneau C. Epidemiologic and socioeconomic burden of metastatic renal cell carcinoma (mRCC): a literature review. Cancer treatment reviews. 2008; 34: 1 93-205.
Nonpatent Reference No. 9: Marks PA, Richon VM, Rifkind RA. Histone deacetylase inhibitors: inducers of differentiation or apoptosis of transformed cells. Journal of the National Cancer Institute. 2000; 92: 1210-6.
Nonpatent Reference No. 10: Koyama M, Izutani Y, Goda AE, et al. Histone deacetylase inhibitors and 15-deoxy-Delta12,14-prostaglandin J2 synergistically induce apoptosis. Clinical cancer research: an official journal of the American Association for Cancer Research. 2010; 16: 2320-32.
Nonpatent Reference No. 11: Hirose T, Sowa Y, Takahashi S, et al. p53-independent induction of Gadd45 by histone deacetylase inhibitor: coordinate regulation by transcription factors Oct-1 and NF-Y. Oncogene 2003; 22: 7762- 73. Nonpatent Reference No. 12: Nakata S, Yoshida T, Horinaka M, et al. Histone deacetylase inhibitors upregulate death receptor 5/TRAIL-R2 and sensitize apoptosis induced by TRAIL/APO2-L in human malignant tumor cells. Oncogene 2004; 23: 6261-71.
Nonpatent Reference No. 13: Yokota T, Matsuzaki Y, Miyazawa K, et al. Histone deacetylase inhibitors activate INK4d gene through Sp1 site in its promoter. Oncogene 2004; 23: 5340-9.
Nonpatent Reference No. 14: Hitomi T, Matsuzaki Y, Yokota T, et al. p15(INK4b) in HDAC inhibitor-induced growth arrest. FEBS Lett 2003; 554: 347-50.
Nonpatent Reference No. 15: Yokota T, Matsuzaki Y, Sakai T. Trichostatin A activates p18INK4c gene: differential activation and cooperation with p19INK4d gene. FEBS Lett 2004; 574: 171-5.
Nonpatent Reference No. 16: Nakano K, Mizuno T, Sowa Y, et al. Butyrate activates the WAF1/Cip1 gene promoter through Sp1 sites in a p53-negative human colon cancer cell line. J Biol Chem 1997; 272: 22199-206.
Nonpatent Reference No. 17: Sowa Y, Orita T, Minamikawa S, et al. Histone deacetylase inhibitor activates the WAF1/Cip1 gene promoter through the Sp1 sites. Biochem Biophys Res Commun 1997; 241: 142-50.
Nonpatent Reference No. 18: Shindoh N, Mori M, Terada Y, et al. YM753, a novel histone deacetylase inhibitor, exhibits antitumor activity with selective, sustained accumulation of acetylated histones in tumors in the WiDr xenograft model. International journal of oncology. 2008; 32: 545-55.
Nonpatent Reference No. 19: Oda K, Stokoe D, Taketani Y, et al. High frequency of coexistent mutations of PIK3CA and PTEN genes in endometrial carcinoma. Cancer Res 2005; 65: 10669-73.
Nonpatent Reference No. 20: Zhao D, Li XP, Gao M, et al. Stromal cell-derived factor 1α stimulates human endometrial carcinoma cell growth through the activation of both extracellular signal-regulated kinase 1/2 and Akt. Gynecol Oncol 2006; 103: 932-7.
Nonpatent Reference No. 21: Gu C, Zhang Z, Yu Y, et al. Inhibiting the PI3K/Akt pathway reversed progestin resistance in endometrial cancer. Cancer Sci 2011; 102: 557-64.
Nonpatent Reference No. 22: Sourbier C, Lindner V, Lang H, et al. The phosphoinositide 3-kinase/Akt pathway: a new target in human renal cell carcinoma therapy. Cancer research. 2006; 66: 5130-42.
Nonpatent Reference No. 23: Brognard J, Clark AS, Ni Y, Dennis PA. Akt/protein kinase B is constitutively active in non-small cell lung cancer cells and promotes cellular survival and resistance to chemotherapy and radiation. Cancer research. 2001; 61: 3986-97.
Nonpatent Reference No. 24: Kulik G, Carson JP, Vomastek T, et al. Tumor necrosis factor alpha induces BID cleavage and bypasses antiapoptotic signals in prostate cancer LNCaP cells. Cancer research. 2001; 61: 2713-9.
Nonpatent Reference No. 25: Izuishi K, Kato K, Ogura T, Kinoshita T, Esumi H. Remarkable tolerance of tumor cells to nutrient deprivation: possible new biochemical target for cancer therapy. Cancer research. 2000; 60: 6201-7.
Nonpatent Reference No. 26: Lee CM, Fuhrman CB, Planelles V, et al. Phosphatidylinositol 3-kinase inhibition by LY294002 radiosensitizes human cervical cancer cell lines. Clinical cancer research: an official journal of the American Association for Cancer Research. 2006; 12: 250-6.
Nonpatent Reference No. 27: Mahalingam D, Medina EC, Esquivel JA, 2nd, et al. Vorinostat enhances the activity of temsirolimus in renal cell carcinoma through suppression of survivin levels. Clinical cancer research: an official journal of the American Association for Cancer Research. 2010; 16: 141-53.
Nonpatent Reference No. 28: Carew JS, Esquivel JA, 2nd, Espitia CM, et al. ELR510444 inhibits tumor growth and angiogenesis by abrogating HIF activity and disrupting microtubules in renal cell carcinoma. PloS one. 2012; 7: e31120.
Nonpatent Reference No. 29: Sosman JA, Puzanov I, Atkins MB. Opportunities and obstacles to combination targeted therapy in renal cell cancer. Clinical cancer research: an official journal of the American Association for Cancer Research. 2007; 13: 764s-9s.

### SUMMARY OF INVENTION

### PROBLEM TO BE SOLVED BY INVENTION

A problem to be solved by the present invention is the provision of new therapeutic agents and treatment strategies permitting treatment and prevention of tumors including malignant tumors such as lung cancer, bronchial cancer, pharyngeal cancer, thyroid cancer, melanoma, breast cancer, bone tumors, soft tissue sarcoma, osteosarcoma, stomach cancer, liver cancer, esophageal cancer, pancreatic cancer, biliary tract cancer, renal cell carcinoma, kidney cancer, adrenal cancer, endometrial cancer, cervical cancer, ovarian cancer, prostate cancer, bladder cancer, large intestine cancer, colon cancer, rectal cancer, skin cancer, mesothelioma, lymphoma, nervous system neoplasms, and nerve tissue neoplasms.

### MEANS FOR SOLVING PROBLEM

With the goal of establishing new, sensible, and highly effective treatment strategies for the foregoing tumors, the present inventor(s) used the molecularly targeted drug PI3K inhibitor LY294002 and the molecularly targeted drug HDAC inhibitor OBP-801, discovered by the present inventor(s) and thought to be effective even for cancers in which there is deactivation of p53, to investigate detailed action in endometrial cancer and renal cell carcinoma, these being examples of tumors for which effective a treatment method has not been established. As a result, it was interestingly and surprisingly discovered that combined use of OBP-801 and PI3K inhibitor provided synergistic cancer therapeutic/prophylactic effect, and through elucidation of the molecular mechanism thereof, the invention of a new tumor therapeutic medication and prophylactic medication based on a new, sensible, and highly effective treatment strategy was completed.

Moreover, to ascertain synergistic tumor therapeutic/preventive effect due to combined use of the discovered OBP-801 and PI3K inhibitor, using, as molecularly targeted drug PI3K inhibitor, BKM120, GDC-0941, BEZ235, BYL719, and CH5132799, these respectively differing from LY294002 in terms of the properties and structures of the compounds, the present inventor(s) determined in all cases that a synergistic tumor therapeutic/prophylactic effect similar to the foregoing was indicated, and moreover, determined that a synergistic effect similar to the foregoing was indicated not only for endometrial cancer and renal cell carcinoma but also for lymphoma and breast cancer, it being determined that the present invention is an invention which is a new tumor therapeutic medication and prophylactic medication, and is an invention which is a tumor treatment method and prevention method, based on a new, sensible, and highly effective treatment strategy.

To enable tumor treatment and prevention, the most essential point of the present invention is that the medication(s) and the treatment/prevention method(s) are such that the molecularly targeted drug HDAC inhibitor OBP-801 and the similarly molecularly targeted drug PI3K inhibitor are used in combination as effective ingredients in medication(s).

Of the means in accordance with the present invention for solving the foregoing problems, a means in accordance with claim 1 is a medical composition for tumor treatment and prevention characterized in that it contains OBP-801 and PI3K inhibitor as effective ingredients.

A means in accordance with claim 2 of the present invention is a medical composition for tumor treatment and prevention which is for use in combination with PI3K inhibitor and which is characterized in that it contains OBP-801 as effective ingredient.

A means in accordance with claim 3 of the present invention is a medical composition for tumor treatment and prevention which is for use in combination with OBP-801 and which is characterized in that it contains PI3K inhibitor as effective ingredient.

A means in accordance with claim 4 of the present invention is a medical composition for tumor treatment and prevention according to any one of claims 1 through 3 characterized in that the PI3K inhibitor is LY294002, BKM120, GDC-0941, BEZ235, BYL719, or CH5132799.

A means in accordance with claim 5 of the present invention is a medical composition for tumor treatment and prevention according to any one of claims 1 through 4 characterized in that the tumor is lung cancer, bronchial cancer, pharyngeal cancer, thyroid cancer, melanoma, breast cancer, bone tumor, soft tissue sarcoma, osteosarcoma, stomach cancer, liver cancer, esophageal cancer, pancreatic cancer, biliary tract cancer, renal cell carcinoma, kidney cancer, adrenal cancer, endometrial cancer, cervical cancer, ovarian cancer, prostate cancer, bladder cancer, large intestine cancer, colon cancer, rectal cancer, skin cancer, mesothelioma, lymphoma, nervous system neoplasm, or nerve tissue neoplasm.

A means in accordance with claim 6 of the present invention is a method for manufacturing a medical composition for tumor treatment and prevention characterized in that it has an operation in which OBP-801 and PI3K inhibitor are made to be contained therein as effective ingredients.

A means in accordance with claim 7 of the present invention is a method for manufacturing a medical composition for tumor treatment and prevention and for use in combination with PI3K inhibitor characterized in that it has an operation in which OBP-801 is made to be contained therein as an effective ingredient.

A means in accordance with claim 8 of the present invention is a method for manufacturing a medical composition for tumor treatment and prevention and for use in combination with OBP-801 characterized in that it has an operation in which PI3K inhibitor is made to be contained therein as an effective ingredient.

A means in accordance with claim 9 of the present invention is a method for manufacturing the medical composition for tumor treatment and prevention according to any one of claims 6 through 8 characterized in that the PI3K inhibitor is LY294002, BKM120, GDC-0941, BEZ235, BYL719, or CH5132799.

A means in accordance with claim 10 of the present invention is a method for manufacturing the medical composition for tumor treatment and prevention according to any one of claims 6 through 9 characterized in that the tumor is lung cancer, bronchial cancer, pharyngeal cancer, thyroid cancer, melanoma, breast cancer, bone tumor, soft tissue sarcoma, osteosarcoma, stomach cancer, liver cancer, esophageal cancer, pancreatic cancer, biliary tract cancer, renal cell carcinoma, kidney cancer, adrenal cancer, endometrial cancer, cervical cancer, ovarian cancer, prostate cancer, bladder cancer, large intestine cancer, colon cancer, rectal cancer, skin cancer, mesothelioma, lymphoma, nervous system neoplasm, or nerve tissue neoplasm.

A means in accordance with claim 11 of the present invention is a medication for tumor treatment, a medication for tumor prevention, and a kit in which there is said medication for tumor treatment and said medication for tumor prevention, comprising a medical composition containing OBP-801 as effective ingredient and a medical composition containing PI3K inhibitor as effective ingredient.

A means in accordance with claim 12 of the present invention is a medication for tumor treatment, the medication for tumor prevention, and the kit in which there is said medication for tumor treatment and said medication for tumor prevention according to claim 11 characterized in that the PI3K inhibitor is LY294002, BKM120, GDC-0941, BEZ235, BYL719, or CH5132799.

A means in accordance with claim 13 of the present invention is a medication for tumor treatment, the medication for tumor prevention, and the kit in which there is said medication for tumor treatment and said medication for tumor prevention according to any one of claims 11 and 12 characterized in that the tumor is lung cancer, bronchial cancer, pharyngeal cancer, thyroid cancer, melanoma, breast cancer, bone tumor, soft tissue sarcoma, osteosarcoma, stomach cancer, liver cancer, esophageal cancer, pancreatic cancer, biliary tract cancer, renal cell carcinoma, kidney cancer, adrenal cancer, endometrial cancer, cervical cancer, ovarian cancer, prostate cancer, bladder cancer, large intestine cancer, colon cancer, rectal cancer, skin cancer, mesothelioma, lymphoma, nervous system neoplasm, or nerve tissue neoplasm.

A means in accordance with claim 14 of the present invention is a tumor treatment and prevention method characterized by administration of OBP-801 and PI3K inhibitor as effective ingredients.

A means in accordance with claim 15 of the present invention is a tumor treatment and prevention method according to claim 14 characterized in that the PI3K inhibitor is LY294002, BKM120, GDC-0941, BEZ235, BYL719, or CH5132799.

A means in accordance with claim 16 of the present invention is a tumor treatment and prevention method according to any one of claims 14 and 15 characterized in that the tumor is lung cancer, bronchial cancer, pharyngeal cancer, thyroid cancer, melanoma, breast cancer, bone tumor, soft tissue sarcoma, osteosarcoma, stomach cancer, liver cancer, esophageal cancer, pancreatic cancer, biliary tract cancer, renal cell carcinoma, kidney cancer, adrenal cancer, endometrial cancer, cervical cancer, ovarian cancer, prostate cancer, bladder cancer, large intestine cancer, colon cancer, rectal cancer, skin cancer, mesothelioma, lymphoma, nervous system neoplasm, or nerve tissue neoplasm.

Furthermore, to solve the foregoing problems, the PI3K inhibitor in the context of the means in accordance with the foregoing claims 1 through 16 may be characterized in that it is selected from among SAR245408/XL147, SAR245409/XL765, BGT226, GS-1101/CAL-101, GDC-0980/RG7422, PX-866, GSK2126458, PF-4691502, PKI-587/PF-05212384, BAY-80-6946, AZD-6482, SF1126, rigosertib/ON 01910.Na, ZSTK474, DS-7423, PA799, MLN1117, GSK2636771, PWT33597 mesylate, IPI-145, AMG319, GDC-0032/RG7604, GDC-0084/RG7666, LY3023414, or AZD8186.

### BENEFIT OF THE INVENTION

In accordance with the present invention, OBP-801 and PI3K inhibitor are effective ingredients, being used in combination in medication(s), as a result of which it is possible to obtain synergistic effect whereby tumor growth is inhibited and apoptosis is induced in tumor cells and tumor tissue, and to achieve tumor treatment and prevention without side effects due to effective ingredients. Moreover, as a result of combining different molecularly targeted drugs such as OBP-801 and PI3K inhibitor, it is possible to simultaneously obtain different marked pharmaceutical benefits which are synergistic, not being obtainable through use of either alone, such as caspase pathway activation, Bim upregulation, increased accumulation of intracellular reactive oxygen species, and survivin and XIAP protein downregulation, making it possible to provide clinically effective and new strategies for treatment/prevention of tumors in the form of effective therapeutic agents and prophylactic agents, or tumor treatment methods and prevention methods, capable of application to a wide variety of tumors, including cancer in which there is PI3K/Akt pathway activation or p53 deactivation, or tumors for which chemotherapy, radiation therapy, hormone therapy, and other such conventional treatment methods have low effectiveness.

### BRIEF DESCRIPTION OF THE DRAWINGS

[FIG. 1] Graph showing cell growth inhibition effect in human type 2 endometrial carcinoma cell line HEC-1-A cells by OBP-801 (Exemplary Study 1). Data represent mean ± SD. ** indicates P < 0.01 (vs. control).
[FIG. 2] Graph showing cell growth inhibition effect in HEC-1-A cells by LY294002 (Exemplary Study 1). Data represent mean ± SD. ** indicates P < 0.01 (vs. control).
[FIG. 3] Results of western blot analysis when HEC-1-A cells were treated with OBP-801 alone (Exemplary Study 1).
[FIG. 4] Results of western blot analysis when HEC-1-A cells were treated with LY294002 alone (Exemplary Study 1).
[FIG. 5] Graph showing inhibitory effect on colony formation due to combinedtreatment of HEC-1-A cells with OBP-801 and LY294002 (Working Example 2). Data represent mean ± SD. ** indicates P < 0.01.
[FIG. 6] Photographs showing inhibitory effect on colony formation due to combined treatment of HEC-1-A cells with OBP-801 and LY294002 (Working Example 2).
[FIG. 7] Results of western blot analysis when HEC-1-A cells were subjected to combined treatment with OBP-801 and LY294002 (Working Example 2).
[FIG. 8] Results of tumor cell cycle progression analysis when HEC-1-A cells were subjected to combined treatment with OBP-801 and LY294002 for 24 hours (Working Example 3). Data represent mean ± SD.
[FIG. 9] Results of Sub-G1 phase cell fraction analysis when HEC-1-A cells were subjected to combined treatment with OBP-801 and LY294002 for 24 hours (Working Example 3). Data represent mean ± SD.
[FIG. 10] Results of cell cycle progression analysis when HEC-1-A cells were subjected to combined treatment with OBP-801 and LY294002 for 48 hours (Working Example 3). Data represent mean ± SD.
[FIG. 11] Results of Sub-G1 phase cell fraction analysis when HEC-1-A cells were subjected to combined treatment with OBP-801 and LY294002 for 48 hours (Working Example 3). Data represent mean ± SD. ** indicates P < 0.01.
[FIG. 12] Results of tumor cell cycle progression analysis when HEC-1-A cells were subjected to combined treatment with OBP-801 and LY294002 for 72 hours (Working Example 3). Data represent mean ± SD.
[FIG. 13] Results of Sub-G1 phase cell fraction analysis when HEC-1-A cells were subjected to combined treatment with OBP-801 and LY294002 for 72 hours (Working Example 3). Data represent mean ± SD. ** indicates P < 0.01.
[FIG. 14] Graph showing cell growth inhibition effect in endometrial carcinoma cell line Ishikawa cells by OBP-801. Data represent mean ± SD. ** indicates P < 0.01.
[FIG. 15] Graph showing cell growth inhibition effect in Ishikawa cells by LY294002. Data represent mean ± SD. ** indicates P < 0.01; * indicates P < 0.05.
[FIG. 16] Results of cell cycle progression analysis when Ishikawa cells were subjected to combined treatment with OBP-801 and LY294002 for 24 hours (Working Example 4). Data represent mean ± SD.

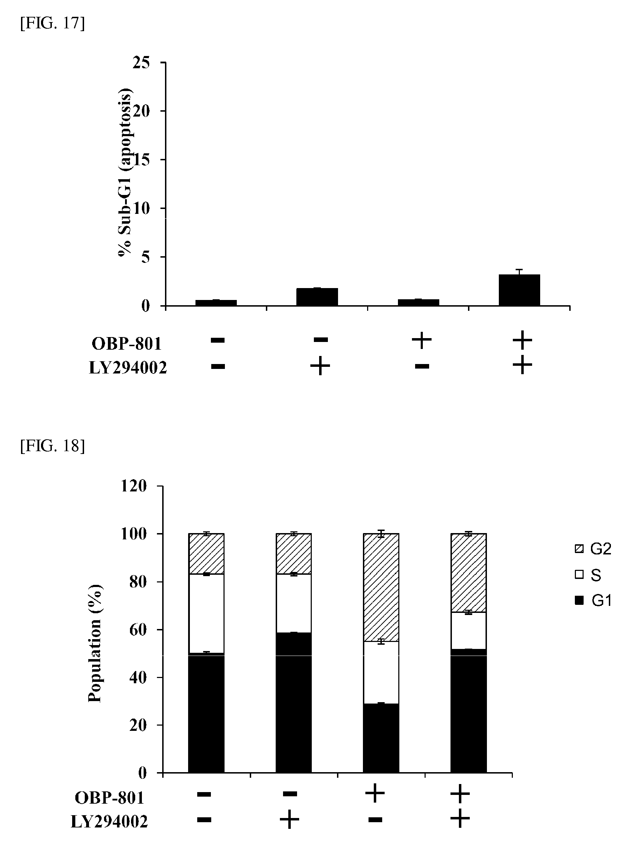

[FIG. 17] Results of Sub-G1 phase cell fraction analysis when Ishikawa cells were subjected to combined treatment with OBP-801 and LY294002 for 24 hours (Working Example 4). Data represent mean ± SD.
[FIG. 18] Results of tumor cell cycle progression analysis when Ishikawa cells were subjected to combined treatment with OBP-801 and LY294002 for 48 hours (Working Example 4). Data represent mean ± SD.
[FIG. 19] Results of Sub-G1 phase cell fraction analysis when Ishikawa cells were subjected to combined treatment with OBP-801 and LY294002 for 48 hours (Working Example 4). Data represent mean ± SD. ** indicates P < 0.01.
[FIG. 20] Graph showing cell growth inhibition effect in renal cell carcinoma cell line 786-O cells by OBP-801. Data represent mean ± SD. * indicates P < 0.05.
[FIG. 21] Graph showing cell growth inhibition effect in 786-O cells by LY294002. Data represent mean ± SD. * indicates P < 0.05.
[FIG. 22] Graph showing cell growth inhibition effect in renal cell carcinoma cell line ACHN cells by OBP-801. Data represent mean ± SD. * indicates P < 0.05.
[FIG. 23] Graph showing cell growth inhibition effect in ACHN cells by LY294002. Data represent mean ± SD. * indicates P < 0.05.
[FIG. 24] Results of viability analysis when 786-O cells were subjected to combined treatment with OBP-801 and LY294002 for 72 hours (Working Example 5). Data represent mean ± SD. * indicates P < 0.05.
[FIG. 25] Results of viability analysis when ACHN cells were subjected to combined treatment with OBP-801 and LY294002 for 72 hours (Working Example 5). Data represent mean ± SD. * indicates P < 0.05.
[FIG. 26] Results of Sub-G1 phase cell fraction analysis when 786-O cells were subjected to combined treatment with OBP-801 and LY294002 for 48 hours and 72 hours (Working Example 5). Data represent mean ± SD. * indicates P < 0.05.
[FIG. 27] Graph analyzing suppression of tumor volume due to administering OBP-801 alone, LY294002 alone, or combination of OBP-801 and LY294002 in xenograft model mice obtained by grafting HEC-1-A onto subcutaneous sites in nude mice (Working Example 6). Data represent mean ± SD. * indicates P < 0.05.
[FIG. 28] Waterfall plot analyzing change in tumor volume due to administering OBP-801 alone, LY294002 alone, or combination of OBP-801 and LY294002 in xenograft model mice obtained by grafting HEC-1-A onto subcutaneous sites in nude mice (Working Example 6).
[FIG. 29] Results of Sub-G1 phase cell fraction analysis to determine whether induction of apoptosis was caspase-dependent when HEC-1-A cells were subjected to combined treatment with OBP-801 and LY294002 (Working Example 7). Data represent mean ± SD. ** indicates P < 0.01.
[FIG. 30] Results of western blot analysis when HEC-1-A cells were subjected to combined treatment with OBP-801 and LY294002 (Working Example 7).
[FIG. 31] Graph to determine whether there was an effect whereby there was increase in accumulation of intracellular reactive oxygen species when HEC-1-A cells were subjected to combined treatment with OBP-801 and LY294002 (Working Example 7). Data represent mean ± SD. * indicates P < 0.05; ** indicates P < 0.01.
[FIG. 32] Results of analysis of relationship between apoptosis induction effect and intracellular reactive oxygen species when HEC-1-A cells were subjected to combined treatment with OBP-801 and LY294002 (Working Example 7). Data represent mean ± SD. ** indicates P < 0.01.
[FIG. 33] Results of western blot analysis when HEC-1-A cells were subjected to combined treatment with OBP-801 and LY294002 (Working Example 8).
[FIG. 34] Results of western blot analysis when HEC-1-A cells subjected to knockdown of Bim by siRNA were subjected to combined treatment with OBP-801 and LY294002 (Working Example 9).
[FIG. 35] Results of Sub-G1 phase cell fraction analysis to determine apoptosis induction effect when HEC-1-A cells subjected to knockdown of Bim by siRNA were subjected to combined treatment with OBP-801 and LY294002 (Working Example 9). Data represent mean ± SD. ** indicates P < 0.01.
[FIG. 36] Results of Sub-G1 phase cell fraction analysis to determine whether induction of apoptosis was caspase-dependent when 786-O cells were subjected to combined treatment with OBP-801 and LY294002 (Working Example 10). Data represent mean ± SD. * indicates P < 0.05.
[FIG. 37] Results of western blot analysis when 786-O cells were subjected to combined treatment with OBP-801 and LY294002 (Working Example 10).
[FIG. 38] Graph to determine whether there was an effect whereby there was increase in accumulation of intracellular reactive oxygen species when 786-O cells were subjected to combined treatment with OBP-801 and LY294002 (Working Example 11). Data represent mean ± SD. * indicates P < 0.05.
[FIG. 39] Results of analysis of relationship between apoptosis induction effect and intracellular reactive oxygen species when 786-O cells were subjected to combined treatment with OBP-801 and LY294002 (Working Example 11). Data represent mean ± SD. * indicates P < 0.05.
[FIG. 40] Results of western blot analysis to ascertain expression of survivin and XIAP when 786-O cells were subjected to combined treatment with OBP-801 and LY294002 (Working Example 12).
[FIG. 41] Results of western blot analysis to ascertain Bcl-2, BAX and Bcl-xL protein expression when 786-O cells were subjected to combined treatment with OBP-801 and LY294002 (Working Example 12).
[FIG. 42] Results of western blot analysis to determine whether downregulation of survivin and XIAP due to combined treatment of 786-O cells with OBP-801 and LY294002 was dependent on generation of intracellular reactive oxygen species (Working Example 12).
[FIG. 43] Results of western blot analysis to ascertain expression of survivin and XIAP when 786-O cells overexpressing survivin and/or XIAP were subjected to combined treatment with OBP-801 and LY294002 (Working Example 13).
[FIG. 44] Results of analysis of effect whereby induction of apoptosis was suppressed when 786-O cells overexpressing survivin and/or XIAP were subjected to combined treatment with OBP-801 and LY294002 (Working Example 13). * indicates P < 0.05.
[FIG. 45] Results of Sub-G1 phase cell fraction analysis to determine apoptosis induction effect when HEC-1-A cells were subjected to treatment in which use of OBP-801 or SAHA was combined with use of LY294002 (Working Example 14). Data represent mean ± SD. ** indicates P < 0.01.
[FIG. 46] Results of Sub-G1 phase cell fraction analysis to determine apoptosis induction effect when 786-O cells were subjected to treatment in which use of OBP-801 or SAHA was combined with use of LY294002 (Working Example 14). Data represent mean ± SD. * indicates P < 0.05.
[FIG. 47] Results of Sub-G1 phase cell fraction analysis to determine apoptosis induction effect when 786-O cells were subjected to treatment with OBP-801 alone or combination with BKM120 (Working Example 15). Data represent mean ± SD. ** indicates P < 0.01.
[FIG. 48] Results of Sub-G1 phase cell fraction analysis to determine apoptosis induction effect when 786-O cells were subjected to treatment with OBP-801 alone or combination with GDC-0941 (Working Example 15). Data represent mean ± SD. ** indicates P < 0.01.
[FIG. 49] Results of Sub-G1 phase cell fraction analysis to determine apoptosis induction effect when 786-O cells were subjected to combined treatment with OBP-801 alone or combination with BEZ235 (Working Example 15). Data represent mean ± SD. ** indicates P < 0.01.
[FIG. 50] Results of Sub-G1 phase cell fraction analysis to determine apoptosis induction effect when 786-O cells were subjected to combined treatment with OBP-801 alone or combination with BYL719 (Working Example 15). Data represent mean ± SD. ** indicates P < 0.01.
[FIG. 51] Results of Sub-G1 phase cell fraction analysis to determine apoptosis induction effect when 786-O cells were subjected to combined treatment with OBP-801 alone or combination with CH5132799 (Working Example 15). Data represent mean ± SD. ** indicates P < 0.01.
[FIG. 52] Results of Sub-G1 phase cell fraction analysis to determine apoptosis induction effect when HEC-1-A cells were subjected to combined treatment with OBP-801 alone or combination with BKM120 (Working Example 16). Data represent mean ± SD. ** indicates P < 0.01.
[FIG. 53] Results of Sub-G1 phase cell fraction analysis to determine apoptosis induction effect when HEC-1-A cells were subjected to combined treatment with OBP-801 alone or combination with GDC-0941 (Working Example 16). Data represent mean ± SD. ** indicates P < 0.01.
[FIG. 54] Results of Sub-G1 phase cell fraction analysis to determine apoptosis induction effect when lymphoma cell line SU-DHL-6 cells were subjected to combined treatment with OBP-801 alone or combination with LY294002 (Working Example 17). Data represent mean ± SD. ** indicates P < 0.01.
[FIG. 55] Results of Sub-G1 phase cell fraction analysis to determine apoptosis induction effect when SU-DHL-6 cells were subjected to combined treatment with OBP-801 alone or combination with BKM120 (Working Example 17). Data represent mean ± SD. ** indicates P < 0.01.
[FIG. 56] Results of Sub-G1 phase cell fraction analysis to determine apoptosis induction effect when SU-DHL-6 cells were subjected to combined treatment with OBP-801 alone or combination with GDC-0941 (Working Example 17). Data represent mean ± SD. ** indicates P < 0.01.
[FIG. 57] Results of Sub-G1 phase cell fraction analysis to determine apoptosis induction effect when breast cancer cell line MDA-MB-231 cells were subjected to combined treatment with OBP-801 alone or combination with LY294002 (Working Example 18). Data represent mean ± SD. ** indicates P < 0.01.
[FIG. 58] Results of Sub-G1 phase cell fraction analysis to determine apoptosis induction effect when MDA-MB-231 cells were subjected to combined treatment with OBP-801 alone or combination with GDC-0941 (Working Example 18). Data represent mean ± SD. ** indicates P < 0.01.

### EMBODIMENTS FOR CARRYING OUT THE INVENTION

The present invention provides a medical composition for tumor treatment and prevention characterized in that it contains, as effective ingredients, a PI3K inhibitor and OBP-801, which is a histone deacetylase (HDAC) inhibitor.

"HDAC inhibitor" as referred to in the present specification indicates a molecularly targeted agent which inhibits histone deacetylase (HDAC) as molecular target.

OBP-801 serving as HDAC inhibitor present within the composition of the present invention may be used by obtaining same from Oncolys Biopharma Inc. (Tokyo, Japan).

HDAC inhibitor SAHA/vorinostat, romidepsin, panobinostat/LBH589, belinostat/PXD101, mocetinostat/MGCD0103, abexinostat/PCI-24781, entinostat/SNDX-275/MS-275, SB939, CS055/HBI-8000, resminostat/4SC-201/BYK408740, givinostat/ITF2357, quisinostat/JNJ-26481585, CHR-2845, CHR-3996, CUDC-101, AR-42, DAC-060, EVP-0334, MGCD-290, CXD-101/AZD-9468, CG200745, arginine butyrate, 4SC-202, rocilinostat/ACY-1215, and SHP-141 may be employed in similar fashion as OBP-801 present within the composition of the present invention.

Furthermore, "PI3K inhibitor" as referred to in the present specification indicates a molecularly targeted agent which inhibits signal transducer PI3K as molecular target.

As the PI3K inhibitor present within the composition of the present invention, LY294002 (2-(4-morpholinyl)-8-phenyl-4H-1-benzopyran-4-one), BEZ235/NVP-BEZ235, SAR245408/XL147, SAR245409/XL765, BGT226, BKM120/NVP-BKM120, GS-1101/CAL-101, GDC-0941/RG7321, GDC-0980/RG7422, PX-866, GSK2126458, PF-4691502, PKI-587/PF-05212384, BAY-80-6946, AZD-6482, SF1126, rigosertib/ON 01910.Na, ZSTK474, DS-7423, PA799, MLN1117, BYL719, GSK2636771, CH5132799, PWT33597 mesylate, IPI-145, AMG319, GDC-0032/RG7604, GDC-0084/RG7666, LY3023414, or AZD8186 may be employed. As the PI3K inhibitor present within the composition of the present invention, LY294002, BKM120, GDC-0941, BEZ235, BYL719, or CH5132799 may be preferably employed.

LY294002 serving as PI3K inhibitor present within the composition of the present invention may be used by obtaining same from Cell Signaling Technology (Beverly, Massachusetts, USA); BKM120, GDC-0941, BEZ235, BYL719, or CH5132799 serving as PI3K inhibitor present within the composition of the present invention may be used by obtaining same from Selleck Chemicals (Houston, Texas, USA) or the like.

In the context of the present invention, the term "tumor" includes benign tumors as well as malignant tumors which may be cancer or other such malignant epithelial tumors or sarcomas or other such malignant non-epithelial tumors. By malignant tumors, this preferably is understood to include lung cancer, bronchial cancer, pharyngeal cancer, thyroid cancer, melanoma, breast cancer, bone tumors, soft tissue sarcoma, osteosarcoma, stomach cancer, liver cancer, esophageal cancer, pancreatic cancer, biliary tract cancer, renal cell carcinoma, kidney cancer, adrenal cancer, endometrial cancer, cervical cancer, ovarian cancer, prostate cancer, bladder cancer, large intestine cancer, colon cancer, rectal cancer, skin cancer, mesothelioma, lymphoma, nervous system neoplasms, and nerve tissue neoplasm tumors; is more preferably understood to include renal cell carcinoma and endometrial cancer; and is still more preferably understood to include endometrial cancer.

In the context of the present invention, "a medical composition for tumor treatment and prevention characterized in that it contains, as effective ingredients, a PI3K inhibitor and OBP-801" means a medical composition in which there is combination of OBP-801 and PI3K inhibitor for simultaneous, separate, or sequential administration in the context of tumor treatment and/or prevention.

The medical composition of the present invention may be provided in the form of a combination drug that contains both OBP-801 and PI3K inhibitor. Furthermore, a medical composition containing OBP-801 and a medical composition containing PI3K inhibitor may be provided as separate medications, and these medical compositions may be used simultaneously, separately, or sequentially. Moreover, it may be provided in the form of a kit consisting of a medication comprising a medical composition containing OBP-801 and a medication comprising a medical composition containing PI3K inhibitor.

Moreover, in accordance with the present invention, when OBP-801 and PI3K inhibitor are used in combination for tumor treatment and prevention, either or both may be administered at a dosage or at respective dosages that is or are lower than would be the case were this or these to be used alone.

The composition of the present invention which contains OBP-801 and PI3K inhibitor contains OBP-801 and PI3K inhibitor as essential ingredients, and may contain additive(s) as desired for the purpose of formulation as a pharmaceutical preparation. It is preferred that OBP-801 and PI3K inhibitor be the only effective ingredients contained therein, and that the medical composition contains additive(s) for the purpose of formulation as a pharmaceutical preparation.

In accordance with the present invention, when it is said that OBP-801 and PI3K inhibitor are contained therein as effective ingredients, this should be understood to mean that OBP-801 and PI3K inhibitor are contained therein as principal active ingredients, but should not be understood as limiting with respect to the content rate of OBP-801 or of PI3K inhibitor.

In the context of the present invention, the term "treatment" means disappearance of tumors or reduction in the number of cells of a tumor, suppression of tumor growth, and/or improvement of various symptoms caused by tumors, as a result of administration of a medical composition associated with the present invention to a test subject. Furthermore, in the context of the present invention, the term "prevention" means preventing increase in number due to resumption of growth of tumor(s) that had decreased and/or preventing resumption of growth of tumor(s) whose growth had been suppressed.

A medical composition for tumor treatment and prevention in accordance with the present invention may be manufactured as a result of performance of operations which cause OBP-801 and PI3K inhibitor to be contained therein as effective ingredients. Furthermore, in accordance with the present invention, this may be manufactured as a result of performance of operations which cause OBP-801 to be contained as an effective ingredient in a medical composition for tumor treatment and prevention for use in combination with PI3K inhibitor, and/or as a result of performance of operations which cause PI3K inhibitor to be contained as an effective ingredient in a medical composition for tumor treatment and prevention for use in combination with OBP-801. Moreover, in addition to the foregoing operations, operations may be added which cause additive(s) to be contained therein for the purpose of formulation as a pharmaceutical preparation.

In the context of the medical composition of the present invention, when this is provided such that OBP-801 and PI3K inhibitor are respectively contained in separate medical compositions, these may be of the same dosage form or of different dosage forms. For example, the two may be mutually different dosage forms, each of which is one among oral formulation, parenteral formulation, injectable formulation, drip formulation, and intravenous drip formulation; or the two may be the same dosage form, which is one among oral formulation, parenteral formulation, injectable formulation, drip formulation, and intravenous drip formulation Furthermore, the foregoing medical composition may have combined therewithin at least one additional different formulation.

The medical composition of the present invention may be in the form of a bulk liquid solution, a liquid suspension, or other such liquid composition; or in the form of a bulk powder, tablet, or other such solid composition. For example, a liquid composition may take the form of a previously filled/measured ampule or syringe, or where this takes the form of a solid composition, this may be in the form of a pill, tablet, capsule, or the like. Amounts of effective ingredients within such formulations are set so as to permit tumor treatment and/or prevention effect to be obtained.

As the foregoing liquid composition, an isotonic solution containing, e.g., physiological saline solution, glucose, and/or other such adjuvant(s); e.g., D-sorbitol, D-mannose, D-mannitol, and sodium chloride, may be cited as examples; and this may be used in combination with suitable solubilizer; e.g., alcohol; more specifically, ethanol and/or polyalcohol; e.g., propylene glycol and/or polyethylene glycol; and/or nonionic surfactant(s); e.g., polysorbate 80 and/or HCO-50. As oily liquid, sesame oil and soybean oil may be cited as examples, and this may be used in combination with solubilizer in the form of benzyl benzoate and/or benzyl alcohol. Furthermore, this is preferably mixed with buffer(s); e.g., phosphate buffer solution and/or sodium acetate buffer solution; soothing agent(s); e.g., procaine hydrochloride; stabilizer(s); e.g., benzyl alcohol and/or phenol; and/or antioxidant(s).

Furthermore, the foregoing solid composition may contain binding agent(s); e.g., microcrystalline cellulose, tragacanth gum, and/or gelatin; excipient(s); e.g., starch and/or lactose; disintegrating agent(s); e.g., alginic acid, Primogel, and/or corn starch; lubricant(s); e.g., magnesium stearate; glidant(s); e.g., colloidal silicon dioxide; sweetener(s); e.g., sucrose and/or saccharin; and/or fragrance(s); e.g., peppermint, methyl salicylate, orange flavoring, and/or other such arbitrary ingredients; and/or compound(s) which is or are substance(s) with similar propertie(s).

The medical composition of the present invention may be administered orally and/or may be administered parenterally by any of various routes of administration, including oral, rectal, percutaneous, subcutaneous, intravenous, intramuscular, and/or intranasal. Where administration is parenteral, examples which may be cited include administration by injection, transnasal administration, pulmonary administration, and transcutaneous administration. As examples of administration by injection, the therapeutic antibodies of the present invention may be administered systemically or locally by, for example, intravenous injection, intramuscular injection, intraperitoneal injection, subcutaneous injection, or the like. Furthermore, administration method and administration dose may be selected as appropriate depending on patent age and symptoms.

Below, although the present invention is described more specifically in terms of working examples, such descriptions should not be interpreted in a manner that would limit the scope of the present invention.

### WORKING EXAMPLES

In the context of tumor treatment and prevention, it was demonstrated that there was marked tumor therapeutic/preventive effect as a result of combined use of OBP-801 and PI3K inhibitor. In the present working example, human endometrial cancer, human renal cell carcinoma, human lymphoma, and human breast cancer were employed as examples of tumors subject to treatment by the present invention; and LY294002, BKM120, GDC-0941, BEZ235, BYL719, and CH5132799, which are of respectively different structure and so forth, were employed as examples of PI3K inhibitor used in combination with OBP-801.

### Materials and Methods

### 1. Cell Culture

HEC-1-A cells derived from the human type 2 endometrial carcinoma cell line were cultured under conditions of 37° C and 5% CO₂ in RPMI medium to which 10% FBS had been added. Ishikawa human endometrial carcinoma cells were cultured under conditions of 37° C and 5% CO₂ in DMEM medium to which 10% FBS had been added. Human renal cell carcinoma cell line 786-O cells were cultured under conditions of 37° C and 5% CO₂ in RPMI 1640 medium to which 10% FBS, 2 mM glutamine, 100 U/mL penicillin, and 100 µg/mL streptomycin had been added. Human renal cell carcinoma cell line ACHN cells were cultured under conditions of 37° C and 5% CO₂ in DMEM medium to which 10% FBS, 4 mM glutamine, 100 U/mL penicillin, and 100 µg/mL streptomycin had been added. Human lymphoma cell line SU-DHL-6 cells were cultured under conditions of 37° C and 5% CO₂ in RPMI 1640 medium to which 10% FBS, 2 mM glutamine, 100 U/mL penicillin, and 100 µg/mL streptomycin had been added. Human breast cancer cell line MDA-MB-231 cells were cultured under conditions of 37° C and 5% CO₂ in RPMI 1640 medium to which 10% FBS, 2 mM glutamine, 100 U/mL penicillin, and 100 µg/mL streptomycin had been added.

### 2. Reagents

OBP-801 was supplied by Oncolys Biopharma Inc. (Tokyo, Japan). LY294002 was purchased from Cell Signaling Technology (Beverly, Massachusetts, USA). SAHA was purchased from Biomol Research Laboratories (Plymouth Meeting, Pennsylvania, USA). N-acetyl-L-cysteine (NAC) was purchased from Nacalai Tesque, Inc. (Japan). The pan-caspase inhibitor zVAD-fmk was purchased from R&D Systems (Minneapolis, Minnesota, USA). BKM120, GDC-0941, BEZ235, BYL719, and CH5132799 were purchased from Selleck Chemicals (Houston, Texas, USA).

### 3. Cell Viability Testing

Cell viability was determined by carrying out measurements at a wavelength of 450 nm with a Viento multi-well spectrophotometer (DS Pharma Biomedical, Japan) using a Cell Counting Kit-8 (Dojindo Laboratories, Japan).

### 4. Colony Formation Assay

Cells were seeded at a density of 200 cells per well in 6-well plates. After culturing these for 24 hours, treatment was carried out for 48 hours at respective concentrations with or without addition of OBP-801 and/or with addition of LY294002. These were then cultured in growth medium for 10 days, and the number of viable colonies was counted.

### 5. Analysis of Cell Cycle and Apoptosis

Cells were treated for 24 hours, 48 hours, or 72 hours at respective concentrations with or without addition of OBP-801 and/or with addition of LY294002, BKM120, GDC-0941, BEZ235, BYL719, or CH5132799, following which cells were harvested. The cells were permeabilized with 0.1% Triton-X100, following which the nuclei were stained with propidium iodide (PI). DNA content was measured using a FACSCalibur™ flow cytometer (Becton-Dickinson, Franklin Lakes, New Jersey, USA), and was analyzed with ModFit LT™ (Verity Software House, Topsham, Maine, USA) and the Cell Quest™ software package (Becton-Dickinson).

### 6. Combination Index

Combination index (CI) was calculated using CalcuSyn software (Biosoft, UK) based on the method of Chou and Talalay. When CI < 1, it is determined that the effect is synergistic; i.e., greater than the expected additive effect. When CI = 1, it is determined that the effect is additive; when CI > 1, it is determined that the effect is antagonistic.

### 7. Evaluation of Antitumor Activity in Xenograft Model

Female Balb/c nu/nu mice (5 weeks of age) were purchased from Shimizu Laboratory Supplies Co., Ltd. (Japan). The HEC-1-A cell line (1×10⁸ cells) was grafted onto the backs of the mice by subcutaneous injection. Tumor volume was calculated using the formula 1/2 x (length) × (width)². When tumor volume reached 100 to 200 mm³, mice were randomly divided into 4 groups (5 mice per group), and treatment was continued. Diluent only (control group), OBP-801 (5 mg/kg), LY294002 (25 mg/kg), or combination of the foregoing was administered to mice 3 times per week for 2 weeks (on the 1st, 4th, 6th, 8th, 10th, and 13th day). OBP-801 was dissolved in 20% hydroxypropyl-β-cyclodextiin/physiological saline solution, and this was injected into the tail vein. LY294002 was dissolved in dimethyl sulfoxide (DMSO)/1 × PBS buffer solution (dilution ratio 1:1; v/v), and this was administered intraperitoneally. Tumor volume was measured 3 times per week (on the 2nd, 5th, 7th, 8th, 11th, and 14th day). On the 14th day, tumors were excised from the euthanized mice. All experiments and procedures were carried out in accordance with guidelines formulated by the Institutional Care Use Committee.

### 8. Western Blot Analysis

Cells were lysed with lysis solution (50 mM Tris-HCl, 1% SDS, 2 µg/mL leupeptin, 2 µg/mL aprotinin, 0.1% 2-mercaptoethanol, and 1 mM phenylmethyl sulfonyl fluoride). Protein extract was loaded onto a polyacrylamide gel, subjected to electrophoresis, and transferred to a nitrocellulose membrane. Blots were blocked in blocking solution (5% skim milk/TBST) for 1 hour at room temperature, and incubated with appropriate primary antibody for 1 hour at room temperature. Signal was detected with an ECL™ Western Blot Analysis System (GE Healthcare, Piscataway, New Jersey, USA) or a Chemi-Lumi One chemiluminescent detection kit (Nacalai Tesque, Inc., Japan). Antibodies used were rabbit polyclonal antibodies; i.e., anti-cleaved caspase antibody, anti-Akt antibody (Cell Signaling Technology), anti-phosphorylated Akt (Ser473) antibody, anti-histone H4 antibody (Abcam, UK), anti-acetylated histone H4 antibody (Millipore, Billerica, Massachusetts), anti-survivin antibody (R&D Systems), anti-caspase-3 antibody (Cell Signaling Technology), anti-Bcl-2 antibody (Abcam, Cambridge, UK), anti-BAX antibody (Santa Cruz Biotechnology, Santa Cruz, California, USA), anti-Bcl-xL antibody (Santa Cruz); rabbit monoclonal antibody; i.e., anti-Bim antibody (Epitomics, San Diego, California, USA); and mouse monoclonal antibodies; i.e., anti-caspase 9 antibody (Cell Signaling Technology or MBL, Japan), anti-caspase-8 antibody (MBL), and anti-β-actin antibody (Sigma, St. Louis, Missouri, USA).

### 9. Measurement of Intracellular Reactive Oxygen Species

Cells were pretreated for 48 h with or without addition of OBP-801 and/or with addition of LY294002, and were thereafter treated with 10 µM 5-(and-6)-chloromethyl- 2',7'-dichlorodihydrofluorescein diacetate, acetyl ester (CM-H₂DCFDA) (Invitrogen, Carlsbad, California, USA). After 30 minutes of incubation with 10 µM CM-H₂DCFDA, cells were collected by trypsinization, were washed with PBS, and were analyzed by flow cytometry using FACSCalibur™ and CellQuest™ software (Becton-Dickinson).

### 10. Small Interfering (si)RNA Transfection

Knockdown of Bim was achieved by transfection with small interfering RNA (siRNA) using Lipofectamine™ RNAiMAX (Invitrogen).

### 11. Plasmid DNA Transfection

pCMV6-XL5, pCMV6-XL5/survivin, and pCMV6-XL5/XIAP plasmids were purchased from OriGene Technologies (Rockville, Maryland, USA). 786-O cells were seeded at 1 x 10⁵ cells/well in 6-well plates without use of antibiotics. After 24 hours, HilyMax transfection reagent (Dojindo Laboratories) was used according to the manufacturer's instructions to transfect 4 µg of plasmid DNA into cells. 4 hours following transfection, medium was replaced with fresh medium, cells were further cultured for 48 hours in accordance with conditions as to whether this was with or without addition of OBP-801 and LY294002, following which cells were harvested.

### Results

### Exemplary Study 1: Inhibition of Growth of Tumor Cells Due to OBP-801 or the PI3K Inhibitor LY294002

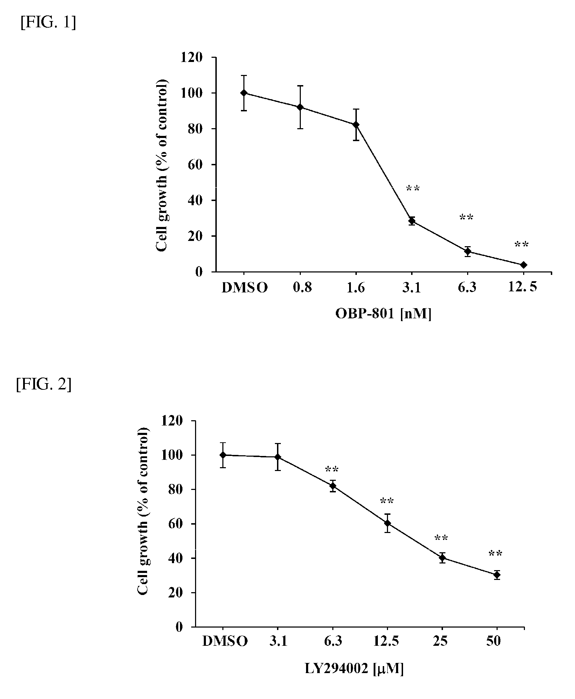

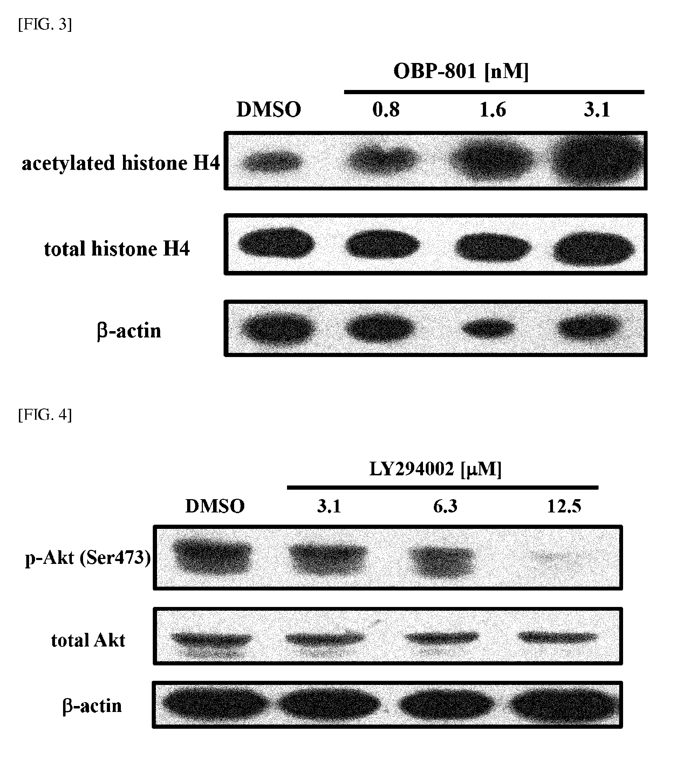

HEC-1-A cells from the human type 2 endometrial carcinoma cell line were used to investigate the effect of OBP-801 or the PI3K inhibitor LY294002 on the growth of tumor cells. As a result, OBP-801 caused inhibition of cell growth at concentrations of 3.1 nM or more, and caused increase of acetylated histone H4 at concentrations of 1.6 nM or more (FIG. 1 and FIG. 3). Furthermore, LY294002 caused inhibition of cell growth, and simultaneously caused decrease in phosphorylated Akt, at concentrations of 6.3 µM or more (FIG. 2 and FIG. 4).

### Working Example 2: Synergistic Enhancement of Inhibitory Effect on Tumor Cell Colony Formation Due to Treatment in which the PI3K Inhibitor LY294002 Is Used in Combination with OBP-801

A colony formation assay employing HEC-1-A cells was carried out to investigate the growth-inhibiting effect of combined treatment with OBP-801 and the PI3K inhibitor LY294002 on tumor cells within tumor tissue. As a result, no reduction in the number of colonies formed was observed even at a concentration as high as 12.5 µM when treatment was carried out using LY294002 alone, but the number of colonies formed was reduced to 60% when treatment was carried out using OBP-801 alone at a concentration of 4 nM.

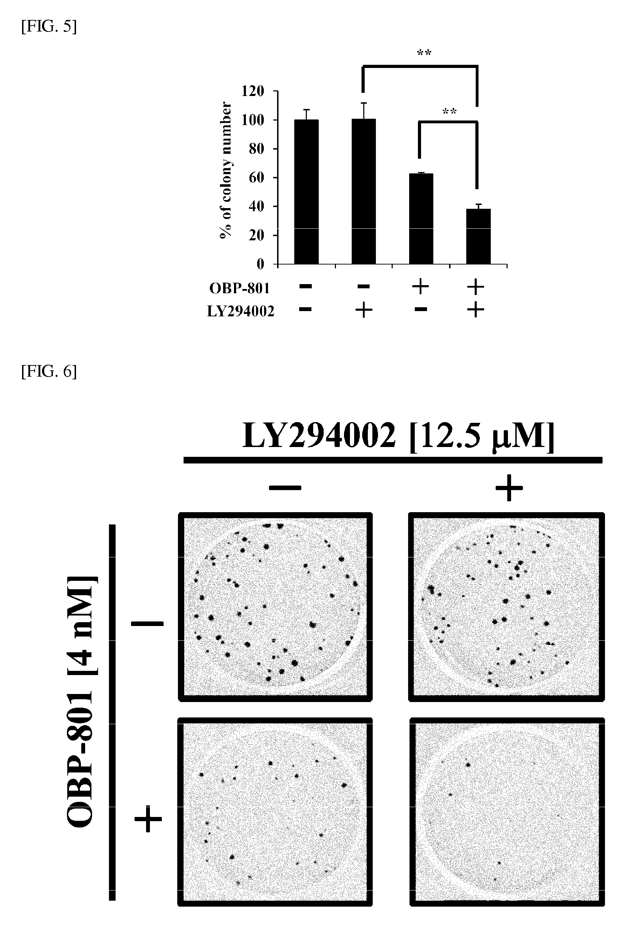

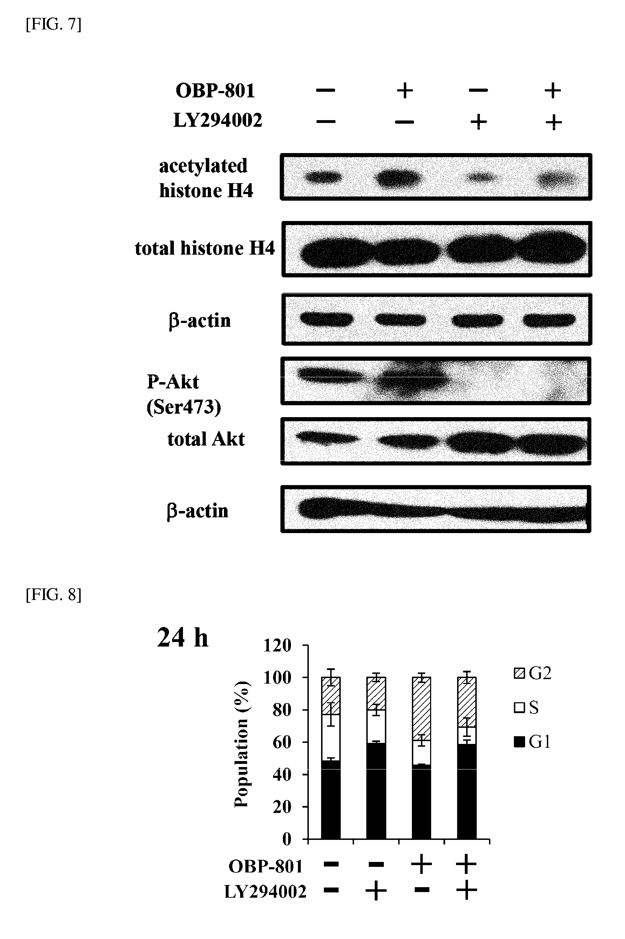

In addition, whereas under the foregoing conditions the PI3K inhibitor LY294002 did not cause decrease in the number of colonies formed when used alone as described above, it was most interestingly discovered that when LY294002 was used in combination with OBP-801 there was indication of an effect whereby inhibition of colony formation by OBP-801 was enhanced (FIG. 5 and FIG. 6). Under the foregoing conditions, increase in acetylated histone H4 and decrease in phosphorylated Akt were observed (FIG. 7).

### Working Example 3: Synergistic Tumor Cell Apoptosis Induction Effect Due to Combined Treatment with OBP-801 and the PI3K Inhibitor LY294002

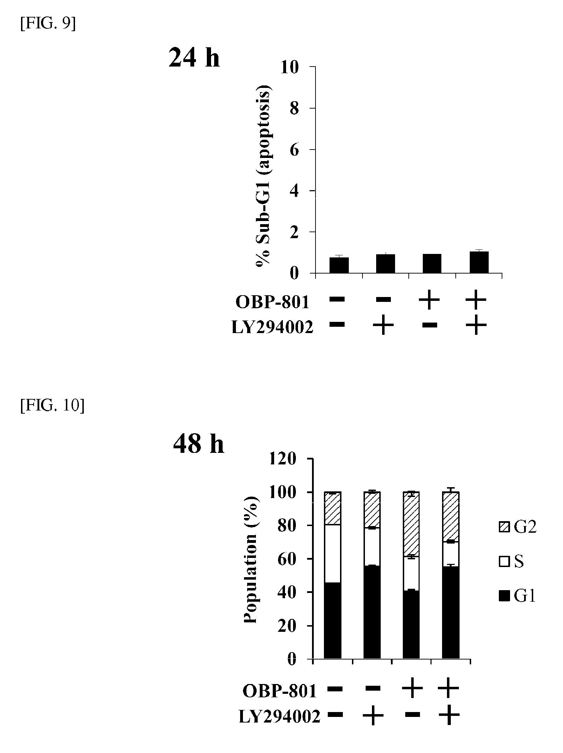

Flow cytometric analysis employing HEC-1-A cells was carried out to investigate whether OBP-801 and the PI3K inhibitor LY294002 had any effect on tumor cell cycle progression. Treatment was carried out using OBP-801 at a concentration of 4 nM, and LY294002 at a concentration of 12.5 µM. As a result, OBP-801 used alone caused G2/M-phase arrest, and LY294002 used alone caused G1-phase arrest, after 24 hours to 72 hours of treatment (FIG. 8, FIG. 10, and FIG. 12).

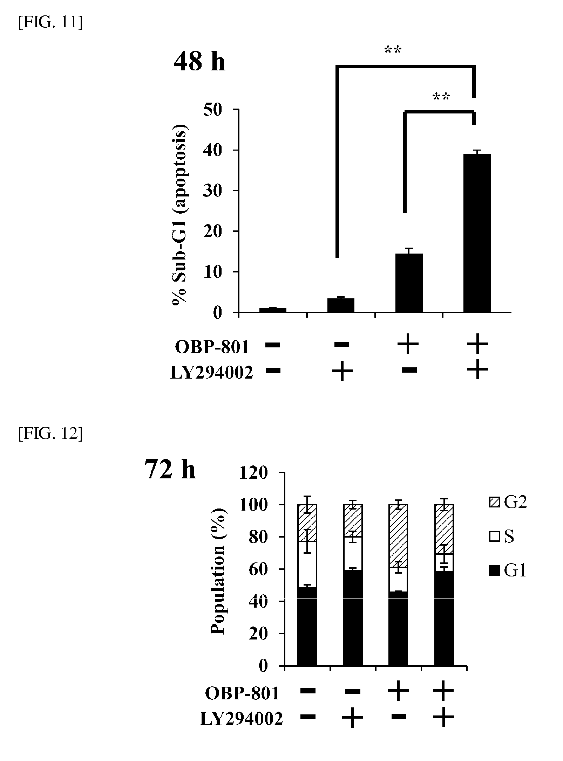

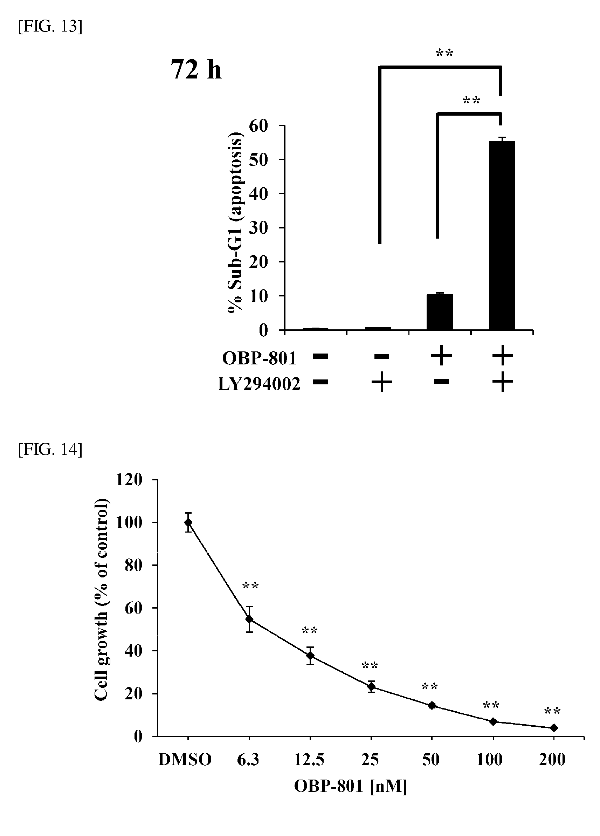

It was however interestingly discovered that OBP-801 and the PI3K inhibitor LY294002 when used in combination caused marked occurrence of induction of apoptosis in HEC-1-A cells after 48 hours to 72 hours of treatment (FIG. 11 and FIG. 13). Moreover, it was determined that combination indices as a result of the foregoing combined treatment were less than 1.0, indicating that the rate of induction of apoptosis was synergistic (TABLE 1).

**[TABLE 1]**

| OBP-801 [nM] | LY294002 [µM] | C.I. [48 h] | C.I. [72 h] |
|---|---|---|---|
| 2 | 3.1 | 2.4 | 7.388 |
| 2 | 12.5 | 0.923 | 0.951 |
| 3 | 3.1 | 0.968 | 1.22 |
| 3 | 12.5 | 0.649 | 0.67 |
| 4 | 3.1 | 0.855 | 0.844 |
| 4 | 12.5 | 0.693 | 0.576 |

Based on these results, it is clear that combined use of OBP-801 and the PI3K inhibitor LY294002 for treatment of cancer cells resulted in synergistic suppression of growth of cancer cells, and moreover, allowed an effect to be obtained whereby there was synergistic induction of apoptosis.

### Working Example 4: Synergistic Tumor Cell Apoptosis Induction Effect Due to Combined Treatment with OBP-801 and the PI3K Inhibitor LY294002

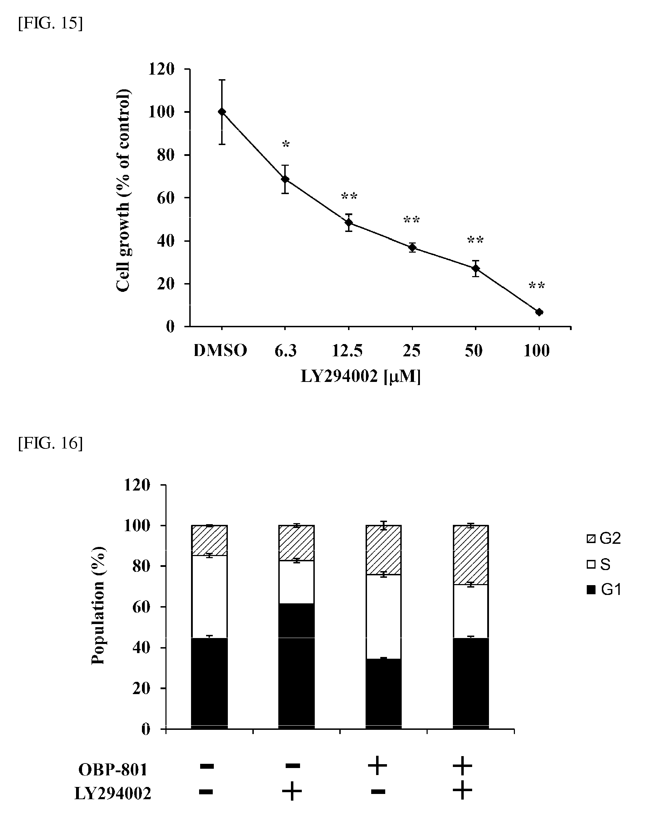

Ishikawa cells from the endometrial carcinoma cell line were used to further investigate the effect of OBP-801 or the PI3K inhibitor LY294002 on the growth of tumor cells. As a result, OBP-801 and LY294002 suppressed growth of Ishikawa cells in concentration-dependent fashion (FIG. 14 and FIG. 15).

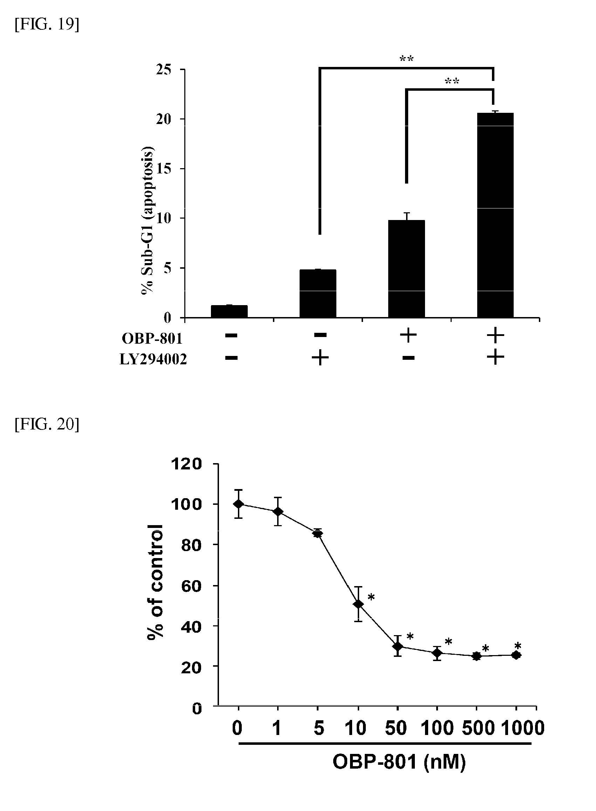

It was however interestingly discovered that, for Ishikawa cells as well, combined use of OBP-801 (6.3 nM) and the PI3K inhibitor LY294002 (12.5 µM) for 48 hours caused synergistic induction of apoptosis which was greater than when either OBP-801 or LY294002 was used alone (FIG. 19).

Based on these results, it is clear that combined use of OBP-801 and the PI3K inhibitor LY294002 produced synergistic cancer cell growth suppression effect and apoptosis induction effect.

### Working Example 5: Synergistic Tumor Cell Growth Inhibition Effect Due to Combined Treatment with OBP-801 and the PI3K Inhibitor LY294002

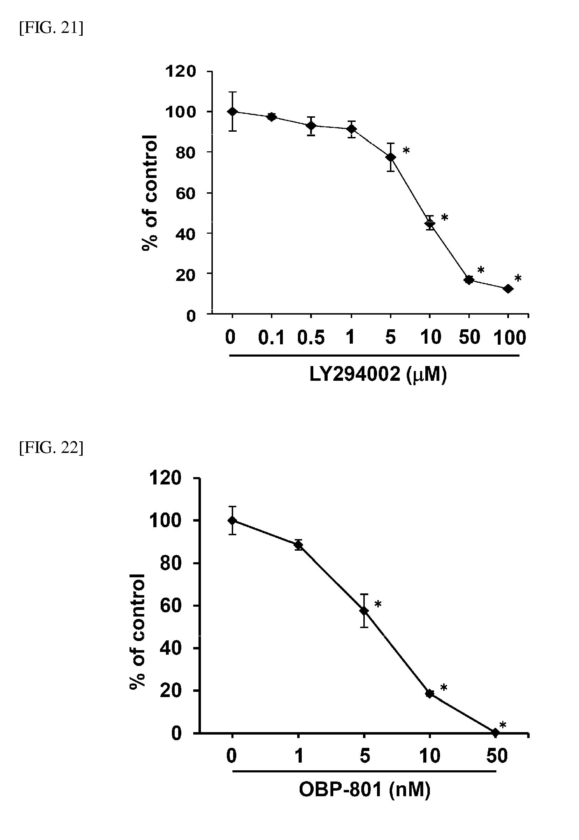

Renal cell carcinoma cell line 786-O cells and renal cell carcinoma cell line ACHN cells were used to further investigate the effect of OBP-801 or the PI3K inhibitor LY294002 on the growth of tumor cells. As a result, OBP-801 and LY294002 suppressed growth of 786-O cells or ACHN cells in concentration-dependent fashion (FIG. 20 through FIG. 23).

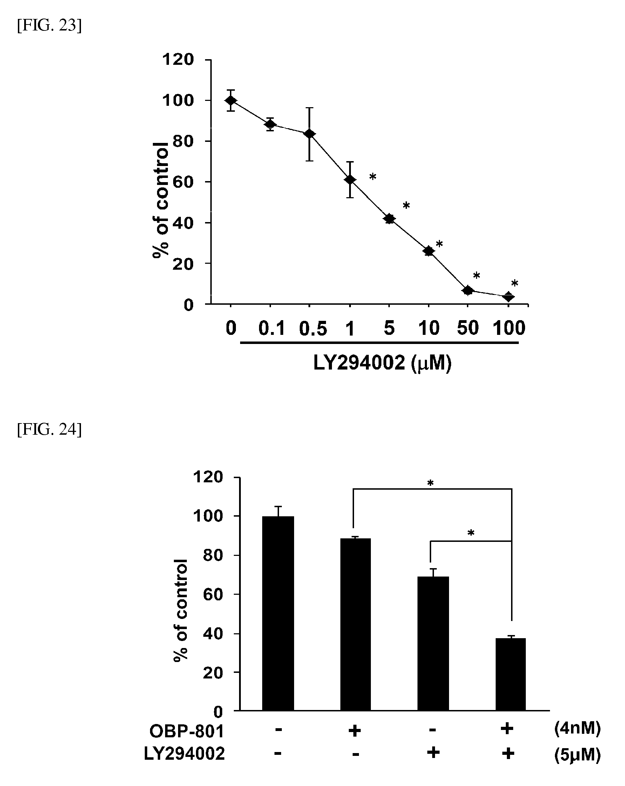

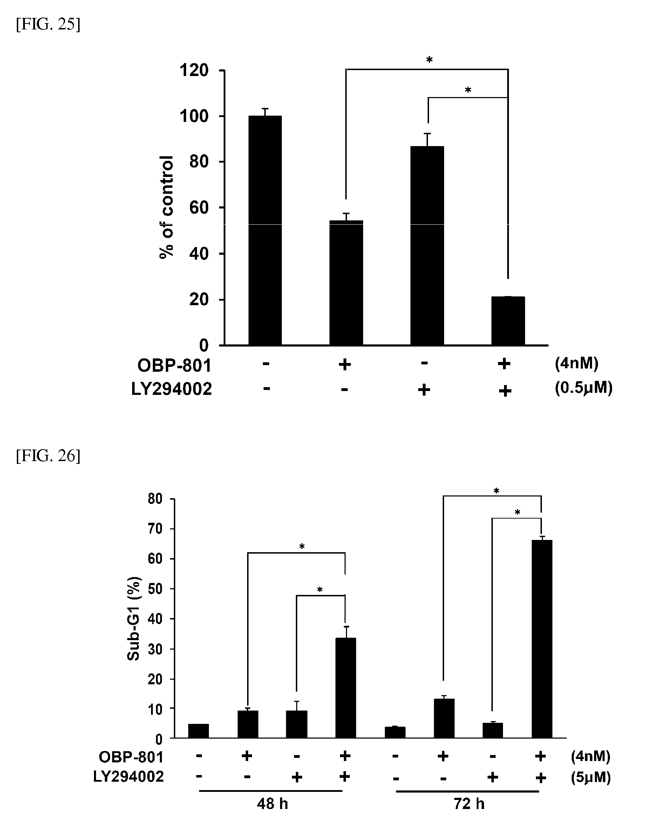

It was however interestingly discovered that, for renal cell carcinoma cells as well, combined use of OBP-801 and the PI3K inhibitor LY294002 at low concentrations caused synergistic suppression of growth of 786-O cells or cells from the ACHN cell line which was greater than when either OBP-801 or LY294002 was used alone (FIG. 24 and FIG. 25). In addition, combination indices as a result of combined treatment with OBP-801 and LY294002 were < 1.0, indicating that there was synergistic tumor cell growth suppression effect (TABLE 2).

**[TABLE 2]**

| OBP-801 | LY294002 | CI |
|---|---|---|
| (nM) | (µM) | |
| 2 | 2.5 | 0.501 |
| 4 | 5 | 0.376 |
| 8 | 10 | 0.381 |
| 16 | 20 | 0.712 |

Moreover, to clarify the mechanism of the synergistic suppression effect due to combined use of OBP-801 and LY294002, the number of 786-O cells in the sub-G1 phase was counted to investigate the effect of combined use on apoptosis. As a result, in the case of the foregoing tumor cells, whereas there was only slight induction of apoptosis as a result of treatment with either OBP-801 or LY294002 alone, it was however interestingly learned that combined treatment with OBP-801 and LY294002 produced synergistic apoptosis induction effect on 786-O cells as well (FIG. 26).

Based on these results, it is clear that combined use of OBP-801 and the PI3K inhibitor LY294002 caused synergistic suppression of growth of tumor cells and induction of apoptosis in tumor cells.

### Working Example 6: Tumor Growth Suppression Effect and Tumor Volume Reduction Effect in Mice Due to Combined Administration of OBP-801 and the PI3K Inhibitor LY294002

OBP-801 and the PI3K inhibitor LY294002, these being molecularly targeted drugs, were administered in combination *in vivo* to investigate whether it was possible to impede growth of tumor cells and tumor tissue within the body of the host animal. Using HEC-1-A cells from the human type 2 endometrial carcinoma cell line as tumor cells, these were grafted onto subcutaneous sites in nude mice, causing the tumor to be carried thereby, to obtain xenograft models. In addition, these were divided into a control group, a group which received administration of OBP-801 alone, a group which received administration of LY294002 alone, and a group which received combined administration of OBP-801 and the PI3K inhibitor LY294002, following which the respective reagents were administered.

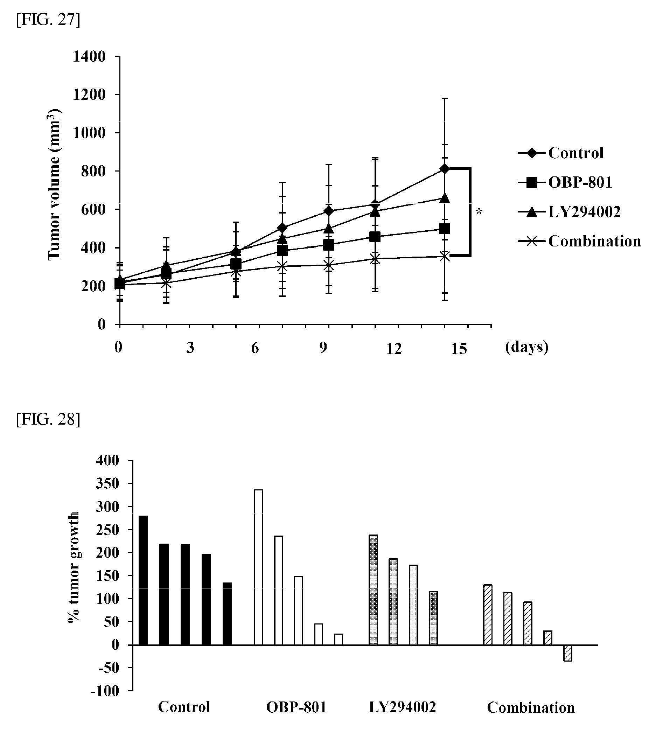

As a result, there was marked suppression of tumor volume in the group received combined administration of OBP-801 and the PI3K inhibitor LY294002 (FIG. 27). Furthermore, not only did combined administration of OBP-801 and the PI3K inhibitor LY294002 produce inhibitory effect on tumor growth, but surprisingly it was also able to reverse tumor growth and bring about reduction thereof (FIG. 28).

Based on these results, it is clear that combined administration of OBP-801 and the PI3K inhibitor LY294002, especially with respect to the point that it was able to reverse tumor growth and bring about reduction thereof, was extremely effective in tumor treatment/prevention.

### Working Example 7: Upregulation of Bim and Caspase-Dependent Induction of Apoptosis Due to Combined Use of OBP-801 and the PI3K Inhibitor LY294002

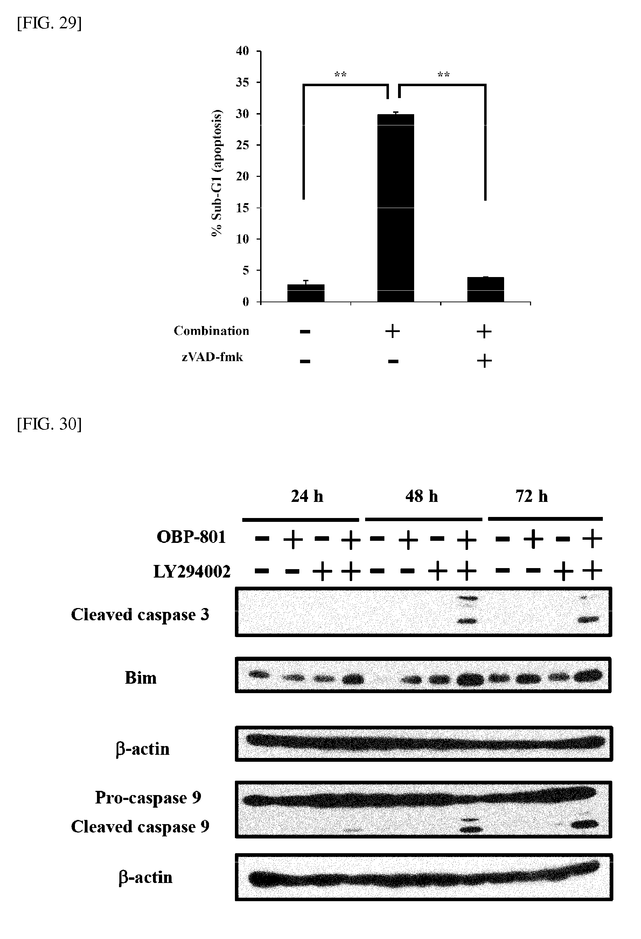

To investigate whether the apoptosis occurring in tumor cells due to combined use of OBP-801 and the PI3K inhibitor LY294002 which was apparent in the foregoing respective working examples was caspase-dependent, a caspase inhibitor was employed to carry out analysis using HEC-1-A cells. As shown in FIG. 29, apoptosis induced by combined use of OBP-801 and PI3K inhibitor was almost completely suppressed by the pan-caspase inhibitor zVAD-fmk. Furthermore, the foregoing combined use clearly promoted caspase cleavage and upregulated Bim, which possesses apoptosis-promoting activity (FIG. 30).

These results suggest that with combined use of OBP-801 and the PI3K inhibitor LY294002 on tumors there is induction of caspase-dependent apoptosis via an intrinsic pathway which includes upregulation of Bim.

### Working Example 8: Increase in Accumulation of Intracellular Reactive Oxygen Species Due to Combined Use of OBP-801 and the PI3K Inhibitor LY294002

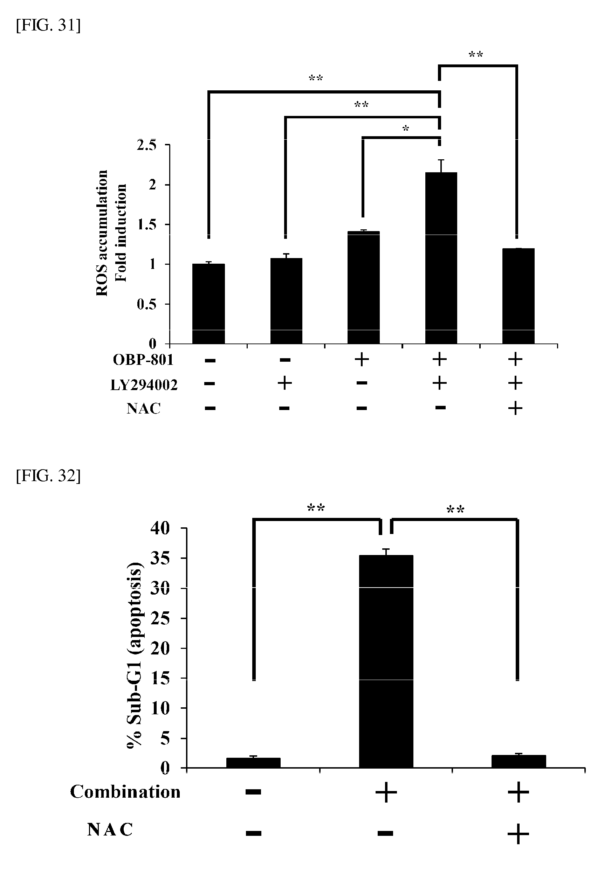

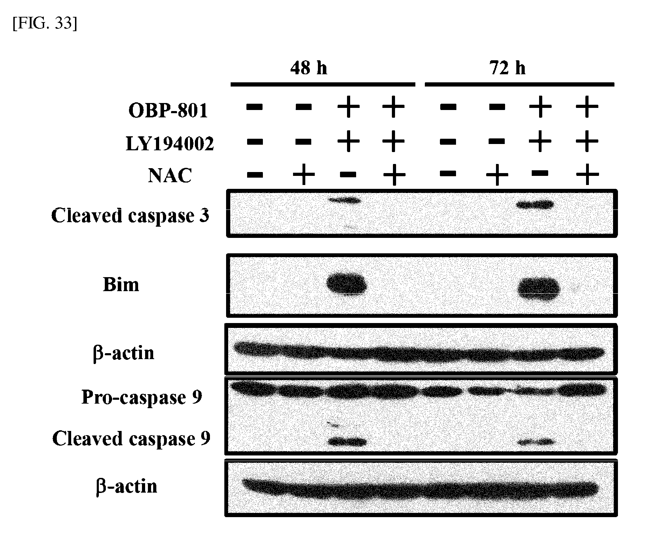

To learn whether the apoptosis induction in tumor cells due to combined treatment with OBP-801 and the PI3K inhibitor LY294002 which was apparent in the foregoing respective working examples was related to intracellular reactive oxygen species, the reactive oxygen species indicator CM-H₂DCFDA was employed to carry out analysis using HEC-1-A cells of any effect whereby intracellular reactive oxygen species might be accumulating in cells treated with OBP-801 and/or LY294002. As a result, combined treatment caused marked increase in accumulation of intracellular reactive oxygen species, and this increase was blocked by N-acetyl-L-cysteine (NAC) (FIG. 31). Moreover, the induction of apoptosis which resulted from combined treatment was in addition almost completely suppressed by NAC (FIG. 32). At the molecular level, NAC inhibited the caspase activation and Bim induction which would otherwise have occurred due to combined treatment (FIG. 33).

These results suggest that induction of apoptosis due to combined treatment with OBP-801 and the PI3K inhibitor LY294002 is mediated by upregulation of Bim occurring as a result of accumulation of intracellular reactive oxygen species.

### Working Example 9: Contribution of Bim Upregulation in Apoptosis Induction Due to Combined Use of OBP-801 and the PI3K Inhibitor LY294002

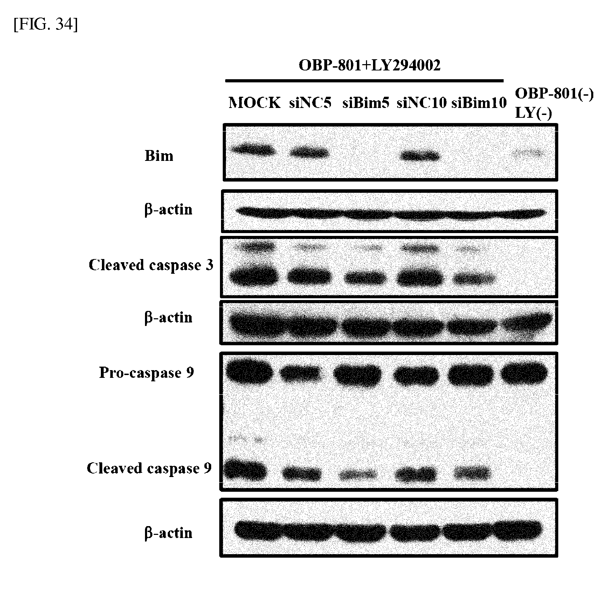

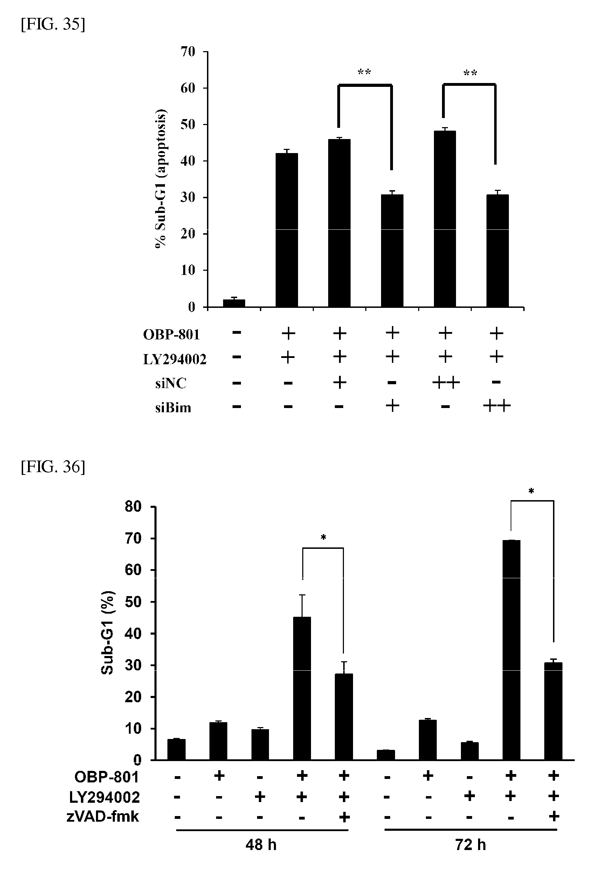

To ascertain the degree to which upregulation of Bim contributed to the induction of apoptosis in tumor cells which was apparent in the foregoing respective working examples, HEC-1-A cells were used to perform knockdown of Bim by siRNA. Knockdown of Bim at least partially suppressed activation of caspases which would otherwise have resulted from combined treatment (FIG. 34), and it was clear that induction of apoptosis due to combined treatment was reduced as compared with control (FIG. 35).

These results suggest that upregulation of Bim at least partially contributes to the enhancement of apoptosis which occurs due to combined treatment with OBP-801 and the PI3K inhibitor LY294002.

### Working Example 10: Caspase-Dependent Induction of Apoptosis Due to Combined Use of OBP-801 and the PI3K Inhibitor LY294002

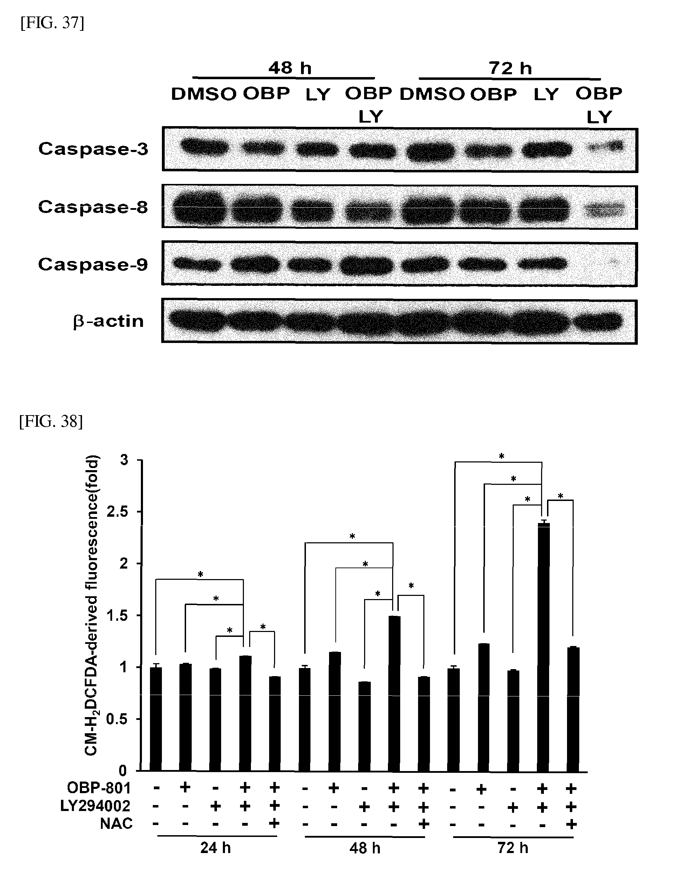

To investigate whether the apoptosis in tumor cells induced due to combined use of OBP-801 and the PI3K inhibitor LY294002 which was apparent in the foregoing respective working examples was dependent on caspases, the pan-caspase inhibitor zVAD-fmk was employed to carry out analysis using 786-O cells. Upon so doing, zVAD-fmk treatment effectively suppressed the apoptosis which would otherwise have been induced as a result of combined treatment with OBP-801 and LY294002 (FIG. 36). Moreover, western blotting was carried out to assess caspase-3, caspase-8 and caspase-9. Whereas treatment with either OBP-801 or LY294002 alone did not induce cleavage of the foregoing caspases, combined treatment with OBP-801 and LY294002 did induce cleavage of the foregoing caspases (FIG. 37).

These results suggest that combined treatment with OBP-801 and the PI3K inhibitor LY294002 induces caspase-dependent apoptosis in 786-O cells.

### Working Example 11: Involvement of Intracellular Reactive Oxygen Species in Tumor Cell Apoptosis Induced as a Result of Combined Treatment with OBP-801 and LY294002

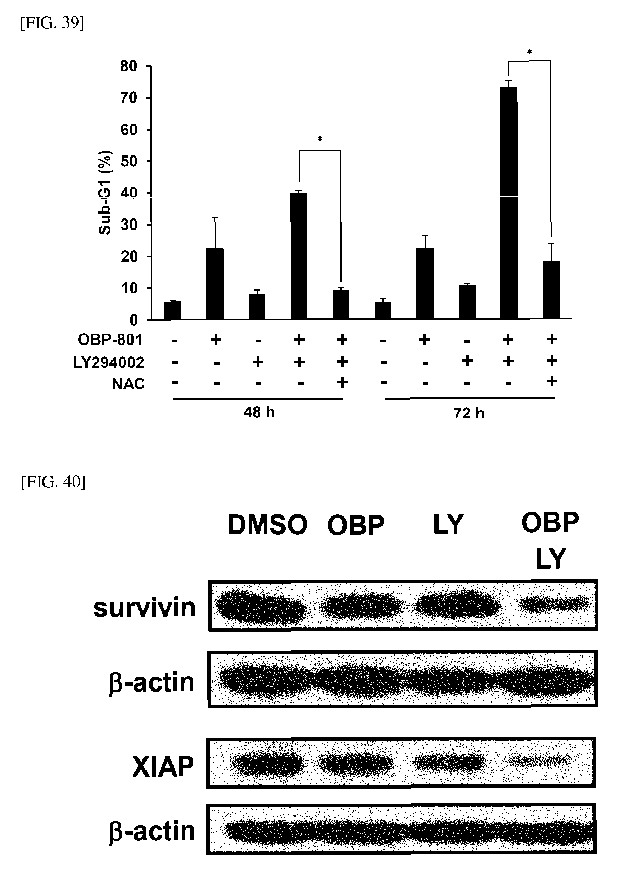

786-O cells were used to investigate whether intracellular reactive oxygen species were involved in the tumor cell apoptosis induced due to combined use of OBP-801 and the PI3K inhibitor LY294002 which was apparent in the foregoing respective working examples. As a result, combined treatment with OBP-801 and LY294002 was able to cause induction of intracellular reactive oxygen species in 786-O cells. Furthermore, treatment with the free radical scavenger NAC blocked the induction of intracellular reactive oxygen species which would otherwise have occurred due to the foregoing combined treatment (FIG. 38). Moreover, NAC also blocked the induction of apoptosis in 786-O cells which would otherwise have occurred due to combined treatment with OBP-801 and LY294002 (FIG. 39).

These results suggest that the induction of apoptosis due to combined treatment with OBP-801 and LY294002 is dependent on generation of intracellular reactive oxygen species.

### Working Example 12: Survivin and XIAP Protein Downregulation through Generation of Intracellular Reactive Oxygen Species in Tumor Cells as a Result of Combined Treatment with OBP-801 and the PI3K Inhibitor LY294002

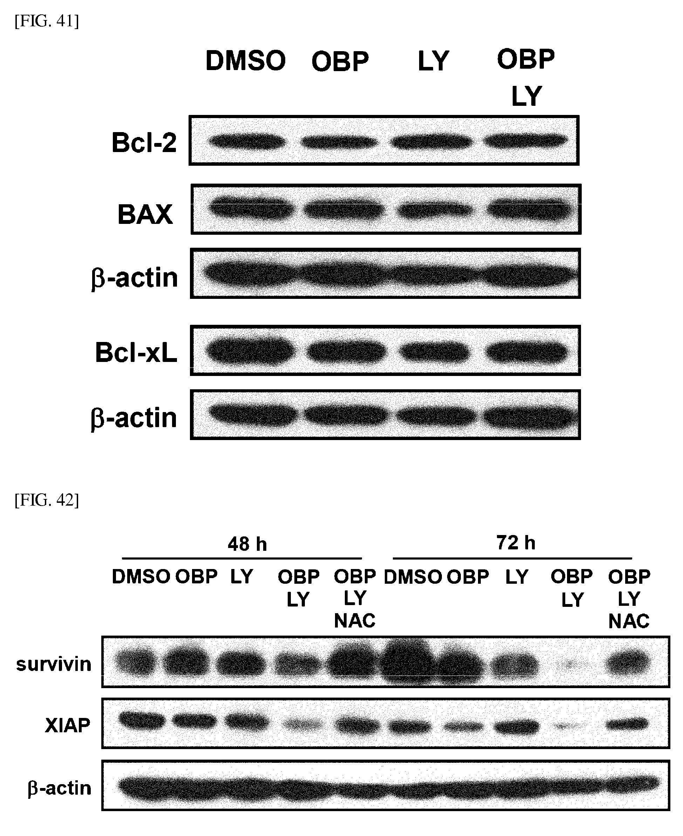

To further explore the molecular mechanism of the apoptosis induction effect in tumor cells due to combined treatment with OBP-801 and the PI3K inhibitor LY294002 which was apparent in the foregoing respective working examples, western blot analysis was carried out using 786-O cells. As shown in FIG. 40, it was found that expression of the anti-apoptotic molecules such as survivin and XIAP was markedly reduced as a result of combined use of OBP-801 and the PI3K inhibitor LY294002 as compared with use of either OBP-801 or LY294002 alone. Bcl-2, BAX and Bcl-xL protein levels were unaffected by combined treatment with OBP-801 and LY294002 (FIG. 41). Investigation was then carried out to determine whether generation of intracellular reactive oxygen species caused downregulation of survivin and XIAP. As shown in FIG. 42, the downregulation of survivin and XIAP was restored by NAC treatment.

These results suggest that the downregulation of survivin and XIAP in tumor cells induced by combined use of OBP-801 and LY294002 is dependent on intracellular reactive oxygen species.

### Working Example 13: Involvement of Survivin and XIAP Downregulation in Apoptosis Induction Due to Combined Use of OBP-801 and the PI3K Inhibitor LY294002

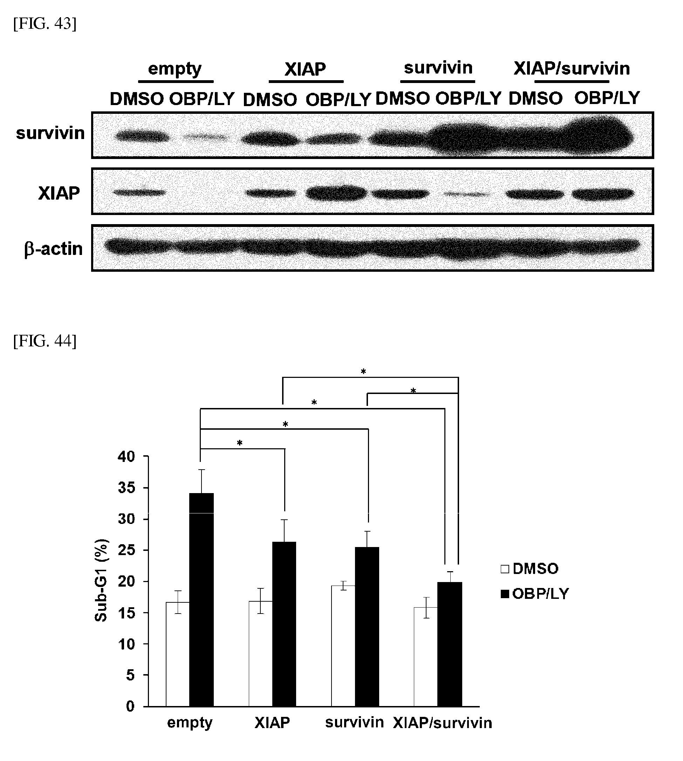

786-O tumor cells were used to investigate whether it was possible by causing overexpression of survivin and XIAP to produce a situation in which there was indication of resistance to the therapeutic combined use of OBP-801 and the PI3K inhibitor LY294002 which was apparent in the foregoing respective working examples. The effects of overexpression of survivin and XIAP were ascertained by western blotting (FIG. 43). In addition, as shown in FIG. 44, it was found that whereas overexpression of survivin or XIAP only partially suppressed induction of apoptosis due to combined use of OBP-801 and LY294002, coexpression of survivin and XIAP satisfactorily suppressed induction of apoptosis due to combined use of OBP-801 and LY294002.

These results suggest that combined use of OBP-801 and the PI3K inhibitor LY294002 causes apoptosis in tumor cells, and that this is at least partially brought about through downregulation of survivin and XIAP.

### Working Example 14: OBP-801 Caused Induction of More Pronounced Apoptosis than SAHA when Used in Combination with LY294002

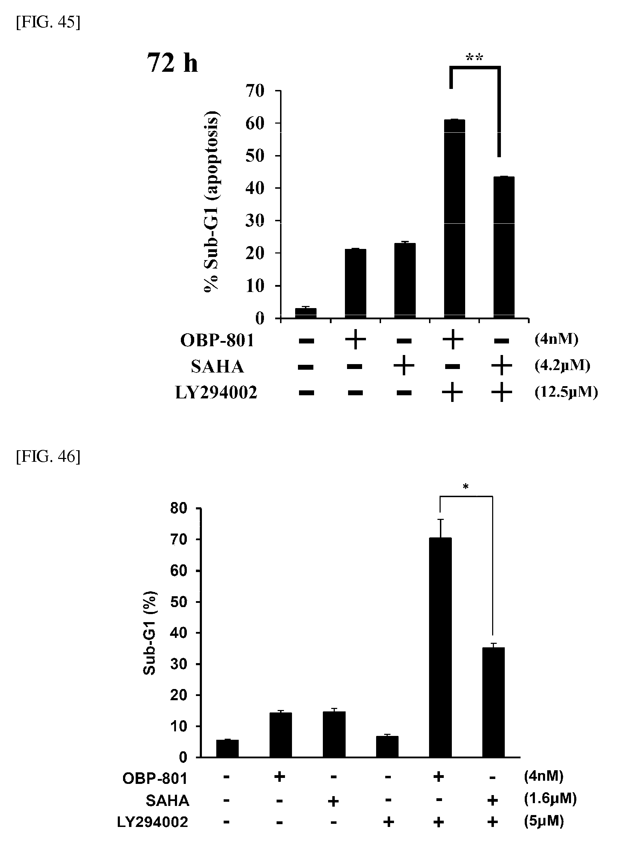

SAHA is the HDAC inhibitor that is most frequently used in clinical contexts. To compare the apoptosis-inducing effect of OBP-801 and SAHA when used in combination with the PI3K inhibitor LY294002, analysis of the sub-G1 phase was carried out by flow cytometry. As shown in FIG. 45 and FIG. 46, whereas apoptosis was induced to approximately the same extent when HEC-1-A cells and 786-O cells were treated with LY294002 alone or were treated with OBP-801 alone, apoptosis as a result of combined treatment with OBP-801 and LY294002, as had been observed in the foregoing respective working examples, was more effectively induced than was the case with combined treatment with SAHA and LY294002.

These results indicate that OBP-801 has more potent action and is more suitable for tumor treatment than SAHA when used in combination with LY294002 in HEC-1-A cells, 786-O cells, or other such tumor cells.

### Working Example 15: Determination of Synergistic Tumor Cell Apoptosis Induction Effect Due to Combined Treatment with OBP-801 and the PI3K Inhibitor BKM120, GDC-0941, BEZ235, BYL719, or CH5132799

Effect exerted on tumor cell growth by OBP-801, PI3K inhibitor, and combined use of OBP-801 and PI3K inhibitor was determined. As tumor cells, renal cell carcinoma cell line 786-O cells were used. As PI3K inhibitor, BKM120, GDC-0941, BEZ235, BYL719, or CH5132799, these being of respectively different structure and so forth, was used. Testing was carried out using procedures similar to those at the foregoing working examples, the fraction of cells in the sub-G1 phase being analyzed 72 hours after addition of reagent was analyzed to determine the apoptosis induction effect of the respective reagent on tumor cells.

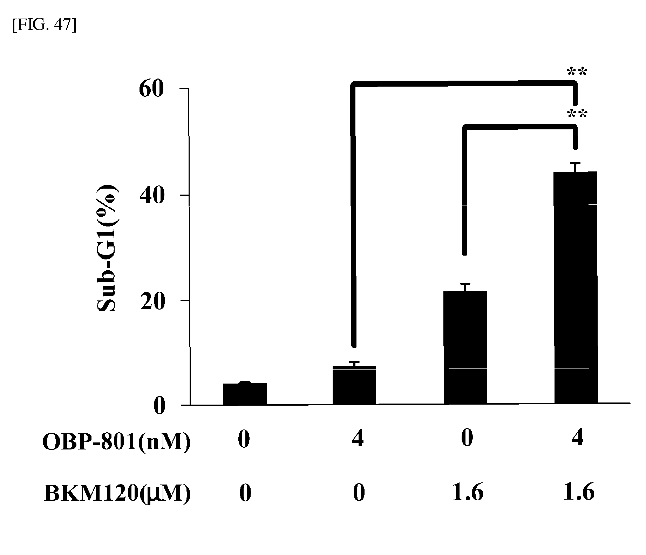

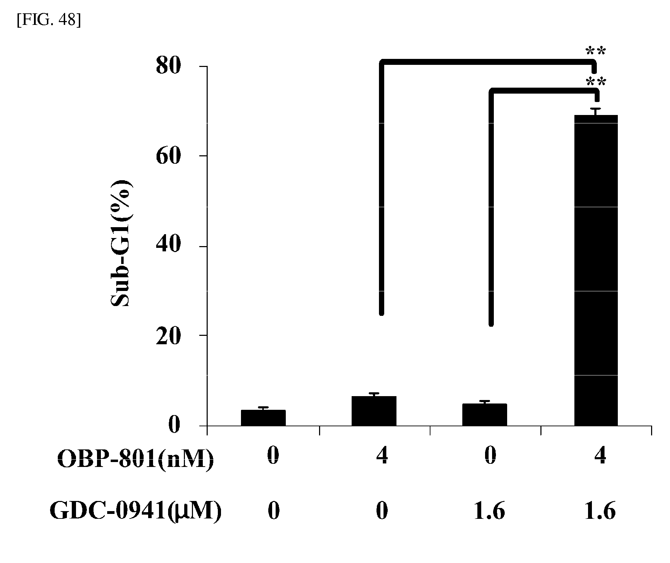

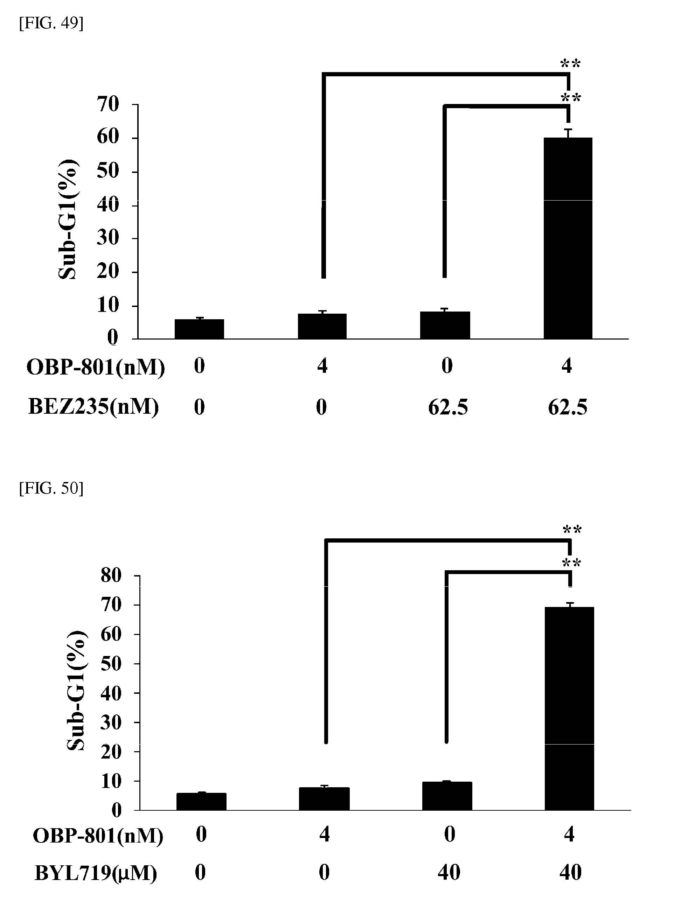

As a result, just as in all of the foregoing cases in which use of PI3K inhibitor was investigated where it was determined that induction of apoptosis was such that combined use with OBP-801 was able to reverse and bring about reduction of growth of tumors placed in mice, it was determined that combined use at concentrations for which no therapeutic/preventive effect was observed when OBP-801 and PI3K inhibitor were used alone was able to provide effect whereby there was synergistic induction of apoptosis in renal cell carcinoma cells (FIG. 47 through FIG. 51). The foregoing tests were repeated twice, and it was determined in each case that it was possible to reproducibly obtain a synergistic apoptosis induction effect.

### Working Example 16: Determination of Synergistic Tumor Cell Apoptosis Induction Effect Due to Combined Treatment with OBP-801 and the PI3K Inhibitor BKM120 or GDC-0941

Effect exerted on tumor cell growth by OBP-801, PI3K inhibitor, and combined use of OBP-801 and PI3K inhibitor was determined. As tumor cells, HEC-1-A cells from the human type 2 endometrial carcinoma cell line were used. As PI3K inhibitor, BKM120 or GDC-0941, these being of respectively different structure and so forth, was used. Testing was carried out using procedures similar to those at the foregoing working examples, the fraction of cells in the sub-G1 phase being analyzed 72 hours after addition of reagent was analyzed to determine the apoptosis induction effect of the respective reagent on tumor cells.

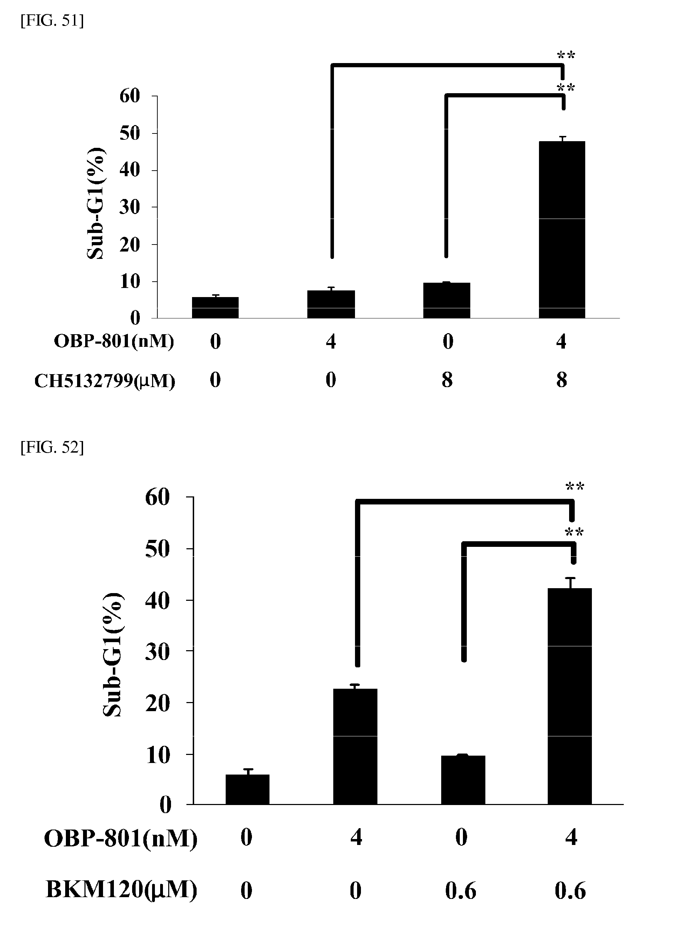

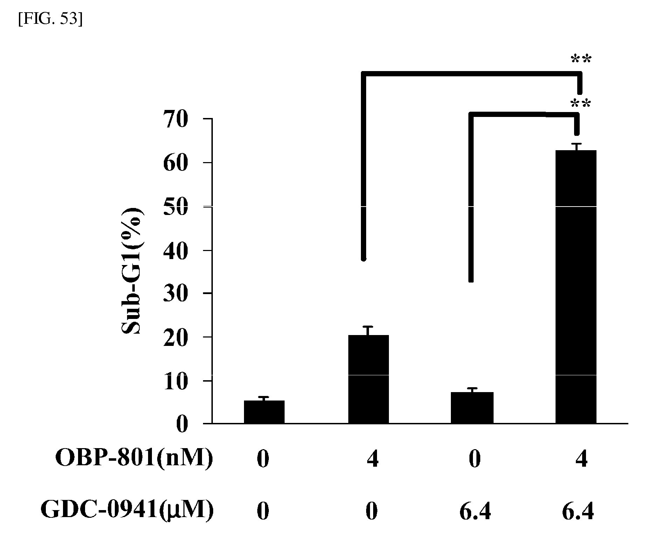

As a result, just as it had been determined that induction of apoptosis was such that combined use with OBP-801 was able to reverse and bring about reduction of growth of tumors placed in mice, it was determined that combined use at concentrations for which no therapeutic/preventive effect was observed when OBP-801 and PI3K inhibitor were used alone was able to provide effect whereby there was synergistic induction of apoptosis in endometrial carcinoma cells (FIG. 52 and FIG. 53). The foregoing tests were repeated twice, and it was determined in each case that it was possible to reproducibly obtain the synergistic apoptosis induction effect due to combined use of OBP-801 and PI3K inhibitor.

### Working Example 17: Determination of Synergistic Tumor Cell Apoptosis Induction Effect Due to Combined Treatment with OBP-801 and the PI3K Inhibitor LY294002, BKM120, or GDC-0941

Effect exerted on tumor cell growth by OBP-801, PI3K inhibitor, and combined use of OBP-801 and PI3K inhibitor was determined. As tumor cells, human lymphoma cell line SU-DHL-6 cells were used. As PI3K inhibitor, LY294002, BKM120, or GDC-0941, these being of respectively different structure and so forth, was used. Testing was carried out using procedures similar to those at the foregoing working examples, the fraction of cells in the sub-G1 phase being analyzed 72 hours after addition of reagent was analyzed to determine the apoptosis induction effect of the respective reagent on tumor cells.

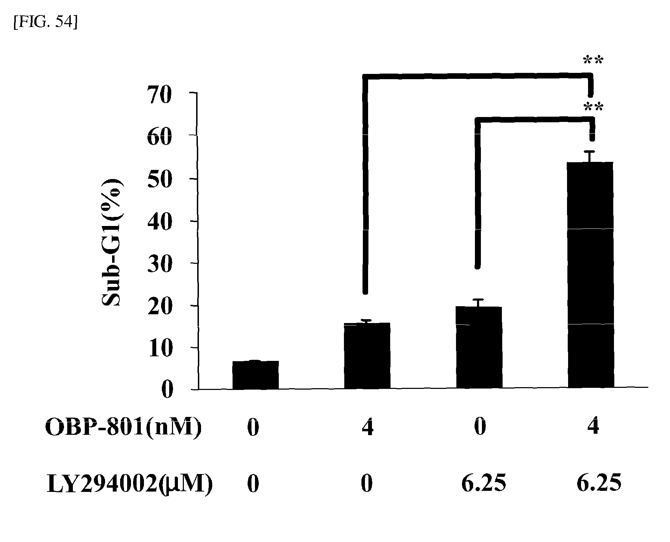

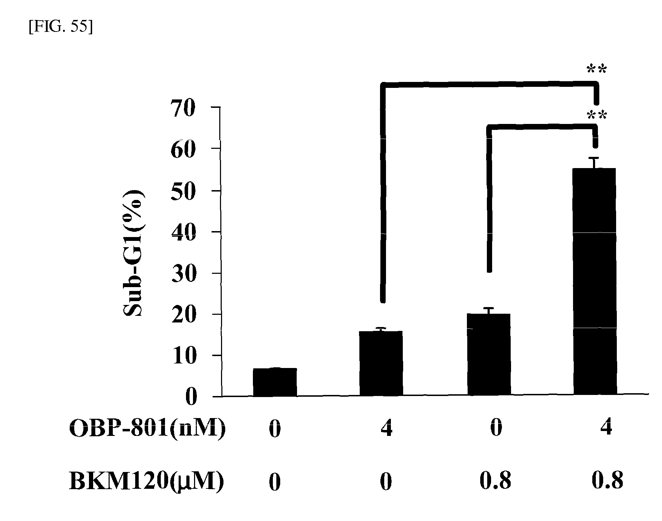

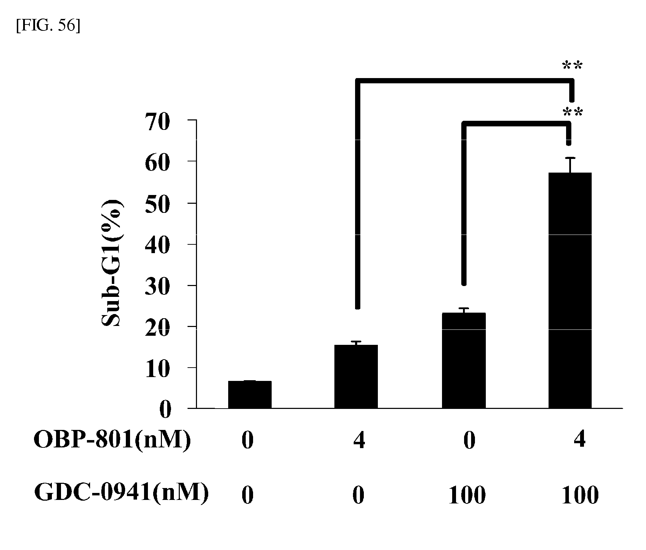

As a result, just as it had been determined that induction of apoptosis was such that combined use with OBP-801 was able to reverse and bring about reduction of growth of tumors placed in mice, it was determined that combined use at concentrations for which no therapeutic/preventive effect was observed when OBP-801 and PI3K inhibitor were used alone was able to provide effect whereby there was synergistic induction of apoptosis in human lymphoma cells as well (FIG. 54 through FIG. 56). The foregoing tests were repeated twice, and it was determined in each case that it was possible to reproducibly obtain the synergistic apoptosis induction effect due to combined use of OBP-801 and PI3K inhibitor.

### Working Example 18: Determination of Synergistic Tumor Cell Apoptosis Induction Effect Due to Combined Treatment with OBP-801 and the PI3K Inhibitor LY294002 or GDC-0941

Effect exerted on tumor cell growth by OBP-801, PI3K inhibitor, and combined use of OBP-801 and PI3K inhibitor was determined. As tumor cells, human breast cancer cell line MDA-MB-231 cells were used. As PI3K inhibitor, LY294002 or GDC-0941, these being of respectively different structure and so forth, was used. Testing was carried out using procedures similar to those at the foregoing working examples, the fraction of cells in the sub-G1 phase being analyzed 72 hours after addition of reagent was analyzed to determine the apoptosis induction effect of the respective reagent on tumor cells.

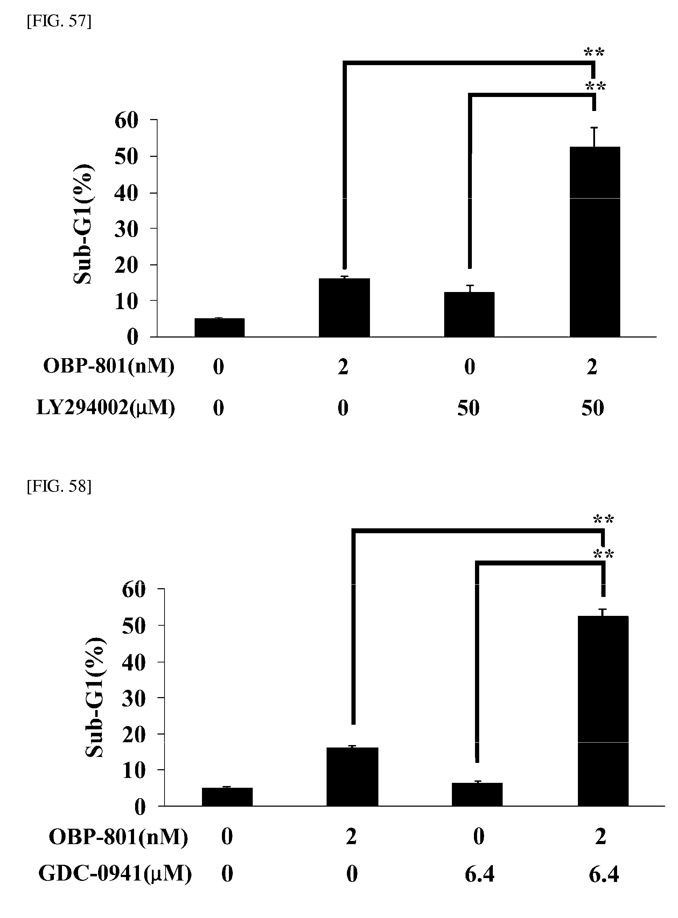

As a result, just as it had been determined that induction of apoptosis was such that combined use with OBP-801 was able to reverse and bring about reduction of growth of tumors placed in mice, it was determined that combined use at concentrations for which no therapeutic/preventive effect was observed when OBP-801 and PI3K inhibitor were used alone was able to provide effect such that there was synergistic induction of apoptosis in the human breast cancer cell line as well (FIG. 57 and FIG. 58). The foregoing tests were repeated twice, and it was determined that it was possible to reproducibly obtain the synergistic apoptosis induction effect due to combined use of OBP-801 and PI3K inhibitor.

### INDUSTRIAL UTILITY

The present invention, in which OBP-801 and PI3K inhibitor are effective ingredients in medication(s) and these are used in combination, makes it possible to obtain synergistic effect whereby tumor growth is inhibited and apoptosis is induced in tumor cells and tumor tissue, and to achieve tumor treatment and prevention without side effects due to effective ingredients. Moreover, as a result of combining different molecularly targeted drugs such as OBP-801 and PI3K inhibitor, it is possible to simultaneously obtain a plurality of different marked pharmaceutical benefits which are synergistic, not being obtainable through use of either formulation alone, such as caspase pathway activation, Bim upregulation, increased accumulation of intracellular reactive oxygen species, and survivin and XIAP protein downregulation, making it possible to provide new and clinically effective tumor treatment/prevention strategies in the form of effective therapeutic agents and prophylactic agents capable of application to a wide variety of tumors, including cancer in which there is PI3K/Akt pathway activation or p53 deactivation, or tumors for which chemotherapy, radiation therapy, hormone therapy, and other such conventional treatment methods have low effectiveness.

## Claims

1. A medical composition for tumor treatment and prevention **characterized in that** it contains OBP-801 and PI3K inhibitor as effective ingredients.

2. A medical composition for tumor treatment and prevention which is for use in combination with PI3K inhibitor and which is **characterized in that** it contains OBP-801 as effective ingredient.

3. A medical composition for tumor treatment and prevention which is for use in combination with OBP-801 and which is **characterized in that** it contains PI3K inhibitor as effective ingredient.

4. The medical composition for tumor treatment and prevention according to any one of claims 1 through 3 **characterized in that** the PI3K inhibitor is LY294002, BKM120, GDC-0941, BEZ235, BYL719, or CH5132799.

5. The medical composition for tumor treatment and prevention according to any one of claims 1 through 4 **characterized in that** the tumor is lung cancer, bronchial cancer, pharyngeal cancer, thyroid cancer, melanoma, breast cancer, bone tumor, soft tissue sarcoma, osteosarcoma, stomach cancer, liver cancer, esophageal cancer, pancreatic cancer, biliary tract cancer, renal cell carcinoma, kidney cancer, adrenal cancer, endometrial cancer, cervical cancer, ovarian cancer, prostate cancer, bladder cancer, large intestine cancer, colon cancer, rectal cancer, skin cancer, mesothelioma, lymphoma, nervous system neoplasm, or nerve tissue neoplasm tumor.

6. A method for manufacturing a medical composition for tumor treatment and prevention **characterized in that** it has an operation in which OBP-801 and PI3K inhibitor are made to be contained therein as effective ingredients.

7. A method for manufacturing a medical composition for tumor treatment and prevention and for use in combination with PI3K inhibitor **characterized in that** it has an operation in which OBP-801 is made to be contained therein as an effective ingredient.

8. A method for manufacturing a medical composition for tumor treatment and prevention and for use in combination with OBP-801 **characterized in that** it has an operation in which PI3K inhibitor is made to be contained therein as an effective ingredient.

9. The method for manufacturing the medical composition for tumor treatment and prevention according to any one of claims 6 through 8 **characterized in that** the PI3K inhibitor is LY294002, BKM120, GDC-0941, BEZ235, BYL719, or CH5132799.

10. The method for manufacturing the medical composition for tumor treatment and prevention according to any one of claims 6 through 9 **characterized in that** the tumor is lung cancer, bronchial cancer, pharyngeal cancer, thyroid cancer, melanoma, breast cancer, bone tumor, soft tissue sarcoma, osteosarcoma, stomach cancer, liver cancer, esophageal cancer, pancreatic cancer, biliary tract cancer, renal cell carcinoma, kidney cancer, adrenal cancer, endometrial cancer, cervical cancer, ovarian cancer, prostate cancer, bladder cancer, large intestine cancer, colon cancer, rectal cancer, skin cancer, mesothelioma, lymphoma, nervous system neoplasm, or nerve tissue neoplasm.

11. A medication for tumor treatment, a medication for tumor prevention, and a kit in which there is said medication for tumor treatment and said medication for tumor prevention, comprising a medical composition containing OBP-801 as effective ingredient and a medical composition containing PI3K inhibitor as effective ingredient.

12. The medication for tumor treatment, the medication for tumor prevention, and the kit in which there is said medication for tumor treatment and said medication for tumor prevention according to claim 11 **characterized in that** the PI3K inhibitor is LY294002, BKM120, GDC-0941, BEZ235, BYL719, or CH5132799.

13. The medication for tumor treatment, the medication for tumor prevention, and the kit in which there is said medication for tumor treatment and said medication for tumor prevention according to any one of claims 11 and 12 **characterized in that** the tumor is lung cancer, bronchial cancer, pharyngeal cancer, thyroid cancer, melanoma, breast cancer, bone tumor, soft tissue sarcoma, osteosarcoma, stomach cancer, liver cancer, esophageal cancer, pancreatic cancer, biliary tract cancer, renal cell carcinoma, kidney cancer, adrenal cancer, endometrial cancer, cervical cancer, ovarian cancer, prostate cancer, bladder cancer, large intestine cancer, colon cancer, rectal cancer, skin cancer, mesothelioma, lymphoma, nervous system neoplasm, or nerve tissue neoplasm.

14. A tumor treatment and prevention method **characterized by** administration of OBP-801 and PI3K inhibitor as effective ingredients.

15. The tumor treatment and prevention method according to claim 14 **characterized in that** the PI3K inhibitor is LY294002, BKM120, GDC-0941, BEZ235, BYL719, or CH5132799.

16. The tumor treatment and prevention method according to any one of claims 14 and 15 **characterized in that** the tumor is lung cancer, bronchial cancer, pharyngeal cancer, thyroid cancer, melanoma, breast cancer, bone tumor, soft tissue sarcoma, osteosarcoma, stomach cancer, liver cancer, esophageal cancer, pancreatic cancer, biliary tract cancer, renal cell carcinoma, kidney cancer, adrenal cancer, endometrial cancer, cervical cancer, ovarian cancer, prostate cancer, bladder cancer, large intestine cancer, colon cancer, rectal cancer, skin cancer, mesothelioma, lymphoma, nervous system neoplasm, or nerve tissue neoplasm.
